# EUROPEAN PATENT SPECIFICATION

(11) **EP 0 752 872 B1**
(45) Date of publication and mention of the grant of the patent: **29.10.2008**
(21) Application number: 95918829.3
(22) Date of filing: 04.04.1995
(51) Int. Cl.: C12Q 1/26

(54) **NADH OXIDASE AS A TARGET IN DIAGNOSIS AND THERAPY**
NADH-OXIDASE ALS ZIELMOLEKÜL IN DIAGNOSE UND THERAPIE
NADH OXYDASE UTILISEE COMME CIBLE DANS DES PROCEDES DE DIAGNOSTIC ET THERAPEUTIQUES

(30) Priority: 05.04.1994 US 222799; 24.05.1994 US 248084
(43) Date of publication of application: 15.01.1997
(73) Proprietor: Morré, D. James, West Lafayette, Indiana 47906 (US); Morré, Dorothy M., Lafayette, Indiana 47906 (US)
(72) Inventor: MORRÉ , D., James, West Lafayette, IN 47906 (US); BYRN, Stephen, R., West Lafayette, IN 47907 (US); CRANE, Frederick, L., West Lafayette, IN 47907 (US); MORRÉ, Dorothy, M., West Lafayette, IN 47906 (US)
(74) Representative: O'Brien, Caroline Jane
(86) International application number: PCT/US1995/004237
(87) International publication number: WO 1995/026743

(56) References cited:
- EP-A- 0 317 957
- EP-A- 0 361 908
- US-A- 4 956 355
- SUN I L ET AL: "INHIBITION OF TRANSPLASMA MEMBRANE ELECTRON TRANSPORT BY TRANSFERRIN ADRIAMYCIN CONJUGATES" BIOCHIMICA ET BIOPHYSICA ACTA, vol. 1105, no. 1, 1992, pages 84-88, XP008036564 ISSN: 0006-3002
- MORRE D J ET AL: "NADH oxidase of liver plasma membrane stimulated by diferric transferrin and neoplastic transformation induced by the carcinogen 2-acetylaminofluorene" BIOCHIMICA ET BIOPHYSICA ACTA - BIOENERGETICS 1991 NETHERLANDS, vol. 1057, no. 1, 1991, pages 140-146, XP008036470 ISSN: 0005-2728
- MORRE D J ET AL: "RETINOIC ACID AND CALCITRIOL INHIBITION OF GROWTH AND NADH OXIDASE OF NORMAL AND IMMORTALIZED HUMAN KERATINOCYTES" BIOCHIMICA ET BIOPHYSICA ACTA, vol. 1134, no. 3, 1992, pages 217-222, XP002299988 ISSN: 0006-3002
- YOSHINARI T ET AL: "REVERSAL OF MULTIDRUG RESISTANCE BY NEW DIHYDROPYRIDINES WITH LOWER CALCIUM ANTAGONISTIC ACTIVITY" CANCER CHEMOTHERAPY AND PHARMACOLOGY, SPRINGER VERLAG, BERLIN, DE, vol. 24, no. 6, 1989, pages 367-370, XP009035799 ISSN: 0344-5704
- MARCILLAT O ET AL: "Oxidative and non-oxidative mechanisms in the inactivation of cardiac mitochondrial electron transport chain components by doxorubicin." THE BIOCHEMICAL JOURNAL. 1 APR 1989, vol. 259, no. 1, 1 April 1989 (1989-04-01), pages 181-189, XP008036571 ISSN: 0264-6021
- JANG J J ET AL: "INHIBITORY EFFECT OF CAPSAICIN IN MOUSE LUNG TUMOR DEVELOPMENT" IN VIVO (ATTIKI), vol. 3, no. 1, 1989, pages 49-54, XP008036660 ISSN: 0258-851X
- SHIMOMURA Y ET AL: "CAPSAICIN AND ITS ANALOGS INHIBIT THE ACTIVITY OF NADH-COENZYME Q OXIDOREDUCTASE OF THE MITOCHONDRIAL RESPIRATORY CHAIN" ARCHIVES OF BIOCHEMISTRY AND BIOPHYSICS, vol. 270, no. 2, 1989, pages 573-577, XP008036627 ISSN: 0003-9861
- MORIMOTO H ET AL: "OVERCOMING TUMOR NECROSIS FACTOR AND DRUG RESISTANCE OF HUMAN TUMORCELL LINES BY COMBINATION TREATMENT WITH ANTI-FAS ANTIBODY AND DRUGS OR TOXINS" CANCER RESEARCH, AMERICAN ASSOCIATION FOR CANCER RESEARCH, BALTIMORE, MD, US, vol. 53, no. 11, 1 June 1993 (1993-06-01), pages 2591-2596, XP000942154 ISSN: 0008-5472
- KIM JOO-HANG ET AL: "Combined use of tamoxifen, cyclosporin A, and verapamil for modulating multidrug resistance in human hepatocellular carcinoma cell lines" YONSEI MEDICAL JOURNAL, vol. 34, no. 1, 1993, pages 35-44, XP002299989 ISSN: 0513-5796
- LEE K ET AL: "EFFICACY OF ANTITUMOR CHEMOTHERAPY IN C3H MICE ENHANCED BY THE ANTIANGIOGENESIS STEROID, CORTISONE ACETATE" CANCER RESEARCH, AMERICAN ASSOCIATION FOR CANCER RESEARCH, BALTIMORE, MD, US, vol. 47, no. 19, 1 October 1987 (1987-10-01), pages 5021-5024, XP001098931 ISSN: 0008-5472
- BRUNO M ET AL: "STIMULATION OF NADH OXIDASE ACTIVITY FROM RAT LIVER PLASMA MEMBRANES BY GROWTH FACTORS AND HORMONES IS DECREASED OR ABSENT WITH HEPATOMA PLASMA MEMBRANES" BIOCHEMICAL JOURNAL, vol. 284, no. 3, 1992, pages 625-628, XP008036459 ISSN: 0264-6021
- FAULK W P ET AL: "TRANSFERRIN ADRIAMYCIN CONJUGATES WHICH INHIBIT TUMOR CELL PROLIFERATION WITHOUT INTERACTION WITH DNA INHIBIT PLASMA MEMBRANE OXIDOREDUCTASE AND PROTON RELEASE IN K562 CELLS" BIOCHEMISTRY INTERNATIONAL, vol. 25, no. 5, 1991, pages 815-822, XP008036457 ISSN: 0158-5231
- JOURNAL OF BIOENERGETICS AND BIOMEMBRANES, Volume 23, Number 4, issued 1991, D. MORRE et al., "NADH Oxidase of Plasma Membranes", pages 469-489.
- BIOCHIMICA ET BIOPHYSICA ACTA, Volume 1057, issued 1991, D. MORRE et al., "NADH Oxidase of Liver Plasma Membrane Stimulated by Diferric Transferrin and Neoplastic Transformation Induced by the Carcinogen 2-Acetylaminofluorene", pages 140-146.
- JOURNAL OF BIOENERGETICS AND BIOMEMBRANES, Volume 26, Number 4, issued August 1994, D. MORRE, "Hormone and Growth Factor Stimulated NADH Oxidase", pages 421-433.
- JOURNAL OF BIOCHEMISTRY, Volume 284, issued 1992, M. BRUNO et al., "Stimulation of NADH Oxidase Activity from Rat Liver Plasma Membranes by Growth Factors and Hormones is Decreased or Absent with Hepatoma Plasma Membranes", pages 625-628.

## Description

### Technical Field

The field of this invention is diagnosis of diseased states and therapeutic treatments of diseased states, using NADH oxidase as the target.

### Background

Enzymatic transfer of electrons from reduced pyridine nucleotide (NADH) to molecular oxygen in the absence of added electron acceptors defines the NADH oxidase activity. This activity is found in isolated plasma membrane vesicles highly purified by aqueous two-phase partition from both animals and plants. The activity is related to growth control and is considered to fulfill a regulatory function as a terminal oxidase of plasma membrane electron support or a related thiol-disulfide exchange. The NADH oxidase activity is reported to be elevated in plasma membranes of carcinogen-induced hyperplastic nodules and hepatomas. In these membranes from neoplastic tissues, the NADH oxidase is apparently no longer subject to growth factor control.

The plasma membrane NADH oxidase differs from mitochondrial oxidases and cellular peroxidases in being insensitive to cyanide. It may account for the stimulation of NADH oxidation by transferrin previously attributed to NADH-diferric transferrin oxidoreductase and to earlier reported NADH oxidations by artificial electron acceptors stimulated by growth factors and hormones. The association of NADH oxidase with growth, its stimulation in normal cells by growth factors, and the loss of this response in neoplastic cells, makes NADH oxidase an interesting target for a determination of its physiological role and the ability to influence that role for therapeutic purposes.

General descriptions of NADH oxidase may be found in Morré et al., Protoplasma 133:195-197 (1986); Brightman et al., Plant Physiol. 86:1264-1269 (1988); Morré et al., Biochim. et Biophys. Acta 1057:140-146 (1991); Morré and Brightman, J. Bioenergetics and Biomembranes 23:469-489 (1991); Brightman et al., ibid, 1105:109-117 (1992); and Bruno et al., Biochem. J. 284:625-628 (1992).

Descriptions of different forms of NADH oxidase and their mechanism of action may be found in Babior, J. Clin. Invest. 73:599-601 (1984); Segal et al., ibid, 83:1785-1793 (1989); Smith and Curnutte, Blood 77:673-686 (1991); Abo et al., Nature 353:668-670 (1991); Nunoi et al., Science 242:1298-1301 (1988); and Volpp et al., Science 242:1295-1297 (1988).

Inhibition of trans-plasma membrane electron support by transferrin-adriamycin conjugates is reported by Sun et al., Biochim. et Biophys. Acta 1105:84-88 (1992).

### SUMMARY OF THE INVENTION

NADH oxidase finds use as a target in the diagnosis of cellular disease states, particularly neoplastic cells and for screening for active agents for the treatment of such diseased states. Particularly, it is found that the NADH oxidase associated with diseased cells is physically and functionally different from NADH oxidase of normal cells. NADH oxidase present in blood circulation may be used as a diagnostic for determining diseased state status. Use of plasma membrane NADH oxidase as a therapeutic target provides for the use of impermeant drugs or drugs conjugated to an impermeant support, which do not require plasma membrane crossing.

Inhibitors of interest include N-Acylated catecholmethylamines, particularly the monomethyl ether. The at least partially purified compounds may be administered to a cell population suspected of having or resulting in neoplastic cells in an amount sufficient to substantially diminish the rate of proliferation of the neoplastic cells. Of particular interest are capsacinoids, where the fatty acid acyl group may be varied as to length, branching and degree of unsaturation, or their conjugates.

The present invention relates in a first aspect to a method for screening agents for their inhibitory effect on plasma membrane NADH oxidase derived from neoplastic cells, said method comprising:
combining in an assay medium said NADH oxidase with said agent in the presence of NADH;
measuring the rate of disappearance of NADH, disappearance of a reactant, or formation of a reaction product as a result of electron transport from said NADH oxidase, and
comparing the inhibitory effect on plasma membrane NADH oxidase derived from neoplastic cells to the inhibitory effect on plasma membrane NADH oxidase from normal cells, wherein said plasma membrane NADH oxidase derived from neoplastic cells and said plasma membrane NADH oxidase from normal cells are physically and functionally different, to identify an inhibitor which discriminates between NADH oxidase from normal cells and NADH oxidase from neoplastic cells.

In a second aspect the present invention relates to a method for determining neoplasia in a mammalian host, said method comprising:
detecting the presence of an NADH oxidase associated with neoplastic cells as compared to a physically and functionally different NADH oxidase associated with normal cells in a biological sample of said host by means of contacting a sample from the mammalian host with an antibody which specifically binds to the NADH oxidase of neoplastic cells but not to the NADH oxidase of normal cells, whereby neoplasia is determined in the mammalian host when said antibody binds to the sample.

In a third aspect, the present invention relates to a method for determining neoplasia in a mammalian host, said method comprising:
combining in an assay medium in the presence of NADH a physiological sample from said host comprising a plasma membrane NADH oxidase and an NADH oxidase inhibitor which discriminates between NADH oxidase from normal cells and NADH oxidase from neoplastic cells, wherein said NADH oxidase from normal cells and NADH oxidase from neoplastic cells are physically and functionally different; and determining the level of said inhibitor at which said NADH oxidase in said sample is inhibited as compared to NADH oxidase from normal cells, where a lower level of inhibitor is indicative of neoplasia.

### BRIEF DESCRIPTION OF THE DRAWINGS

Figure 1 is a graph showing the inhibition of plasma membrane NADH oxidase activity from RLT-N hepatoma by adriamycin, where the ED₅₀ for inhibition is approximately 1 µM; and the inhibition of plasma membrane NADH oxidase from HeLa cells (human ovarian carcinoma) by adriamycin, where ED₅₀ is approximately 0.7 µM.
Figure 2 is a graph showing the inhibition of NADH oxidase activity from isolated plasma membranes of HeLa cells by an acetogenin, bullatacin. The inhibition was measured over the first 5 min of bullatacin addition. Inhibition by bullatacin is time dependent and much stronger inhibitions may be achieved with longer inhibitions (up to 20 min) or by accelerating bullatacin binding to the oxidase by the addition of 10 nM EGF.
Figure 3 is a graph showing the inhibition of NADH oxidase activity from plasma membranes prepared from HeLa cells and from rat liver as a function of concentration of the quassanoid, (-)-glaucarubolone. The activity was 50% inhibited at a final concentration of 0,1 nM with HeLa plasma membranes and at about 1 nM with liver plasma membranes.
Figure 4 is a graph of NADH oxidase activity as a function of the concentration of adriamycin conjugated to Fe₂ transferrin-adriamycin, comparing plasma membranes from cells susceptible (open circles) or resistant (solid circles) to adriamycin.
Figure 5 is graph of the dose response of NADH oxidase from HeLa cell plasma membranes to capsaicin in DMSO (solid circles) or an equivalent amount of DMSO alone (open circles). The response of rat liver plasma membrane vesicles to capsaicin is shown by the open triangles. Values are duplicate determinations plus/minus mean average deviations.
Figure 6 is graph of the dose response of NADH oxidase from HeLa cell plasma membranes to the sulfonylurea LY237868 (solid circles) or a conjugate of LY237868 and α-cyclodextrin (open circles). Values are duplicate determinations plus/minus mean average deviations.
Figure 7 is a graph showing the growth of HeLa cells in the presence of the sulfonylurea LY181984, LY237868, or an LY237868-α-cyclodextrin conjugate.
Figures 8A and 8B show the structure of capsaicin-α-cyclodextrin conjugates with the tail and head of capsaicin, respectively.
Figure 9 is a graph showing the growth of HeLa cells after 72 hours in the presence of capsaicin, or a T-conjugate of capsaicin, as shown in Figure 8A.
Figure 10 is a graph showing the growth inhibition of HeLa cells in the presence of chloroaniline conjugated to α-cyclodextrin, free chloroaniline, or the antitumor sulfonylurea LY 181984.

### DESCRIPTION OF SPECIFIC EMBODIMENTS

In accordance with the subject invention, NADH oxidase is used as a target for a variety of purposes in diagnosis and screening. Particularly, it is found that NADH oxidase differs as to its chemical and biological properties, depending upon the status of the cell from which it is derived. NADH oxidase produced by diseased cells, such as neoplastic cells, can be distinguished from NADH oxidase from normal cells and biologically by its response to a variety of agents.

The NADH oxidase may be from any animal source, particularly mammalian source, such as avian, domestic or laboratory animals, such as murine, feline, canine, porcine, bovine, lagomorpha, ovine, primate, and particularly human. In addition, NADH oxidase may be prepared by recombinant techniques.

The NADH oxidase is useful as a diagnostic tool where cells are suspected of being neoplastic. As such, tissue isolated by biopsy, or NADH oxidase present in the circulation may be detected. Detection may be achieved immunologically, employing antibodies that distinguish between the NADH oxidase of normal cells and diseased cells. Alternatively, detection may be based on the altered drug response of the tumor associated NADH oxidase.

Antibodies specific for the disease associated NADH oxidase are prepared using conventional methods, by immunizing an appropriate mammalian host with the NADH oxidase, the extracellular portion, or portions thereof, to activate an immune response for production of antibodies. See, Antibodies: A Laboratory Manual, eds. Ed Harlow, David Lane Cold Spring Harbor Laboratories, Cold Spring Harbor, NY., 1988. Conveniently, purified proteins are obtained and used as the immunogen, by themselves, or in conjunction with adjuvants, such as alum, complete Freund's adjuvant, mycobacterial fragments, *etc*. By employing mice or other mammalian hosts that allow for fusion of splenocytes with myelomas, or other modes of immortalization, *e*.*g*. viruses or oncogenes, cells may be isolated, expanded and screened for the production of antibodies specific for disease associated NADH oxidase. The immortalized cells may then be expanded, grown as ascites or in culture, and the antibodies harvested.

A wide variety of immunoassays are known, where antibodies are available which are specific for a particular analyte. Thus, the antibodies may be conjugated with a wide variety of labels, e.g. radioisotopes, enzymes, fluorescers, chemiluminescers, or the like, where the label provides a detectable signal. The particular manner in which the NADH oxidase is detected is not critical to this invention and any convenient assay having the necessary sensitivity may be employed.

Alternatively, one may differentiate between the different NADH oxidases by virtue of their different response to a variety of agents. In this mode, one performs an assay with a sample suspected of being associated with a diseased state. The physiological sample may be a tissue sample, plasma membrane fragments, lysate, serum, or other convenient physiological fluid. Conventional assays may be employed for NADH oxidase, where cyanide may be included in the medium to inhibit mitochondrial oxidase activity. The enzyme activity may be followed by the changing concentration of NADH or an electron acceptor which provides for a detectable signal. The NADH oxidase may be further characterized by its protein disulfide-thiol interchange activity, where inhibition of the interchange activity may be measured in response to agents that inhibit the NADH oxidase.

Various agents may be used that will discriminate between NADH oxidase from diseased cells and normal cells. Included among these agents are adriamycin; adriamycin conjugates, *e.g*. transferrin, lactotransferrin, vasopressin, glucagon, or other NADH oxidase stimulator; capsaicin and capsaicin derivatives, *etc.* Thus, by adding an amount of inhibitor which distinguishes between NADH oxidase from diseased cells and NADH oxidase from normal cells, one can assay for the presence of diseased cells. Particularly, adriamycin may be used in concentrations below about 0.5 µM, generally below about 0.2 µM, where depending upon the particular diseased cells, the ED₅₀ of adriamycin may vary from about 0.1 to 1 nM.

For confirmation of the neoplastic state, one may use factors that enhance activity for NADH oxidase from normal cells, but have substantially no effect on NADH oxidase from abnormal cells. Thus, factors such as epidermal growth factor (EGF), transferrin, pituitary extract, lactotransferrin, vasopressin and glucagon may be added to the assay medium in concentrations ranging from about 0.05 to 0.5 µg/ml and the effect of the additive determined. (See Bruno et al., Biochem. J. (1992) 284, 625-628.) If desired, one may perform the assay with a combination of an inhibitor and factor. It is found that with a combination of EGF in a fixed amount and of adriamycin in varying amounts, the result is biphasic, where at low concentrations, below about 10⁻⁶ M, adriamycin is inhibitory, while with concentrations exceeding 10⁻⁶, adriamycin stimulates normal cells. The growth factor has substantially no effect on the enzyme activity from neoplastic cells. However, the NADH oxidase from neoplastic cells appears to have a dose-response to adriamycin similar to that observed with factor activated NADH oxidase from normal cells. Finally, NADH oxidase from normal cells is resistant to inhibition by adriamycin with an ED₅₀ of about 7 µM, in contrast to NADH oxidase from diseased cells, where the adriamycin ED₅₀ for inhibition is about 0.7 nM.

In carrying out the assays, a control will generally be used, where the control is NADH oxidase from normal cells. By comparing the results obtained with NADH oxidase from normal cells, with the results obtained with the sample comprising NADH oxidase, a difference in response as described above may be attributed to neoplasia.

Of particular interest is employing an electron acceptor in the assay for NADH oxidase, conveniently ascorbate radical, where one may follow the rate of disappearance of the ascorbate radical under the conditions of the assay. One can measure the reduction of ascorbate free radical at 265 nm employing a spectrophotometer as described by Winkler (1987) Biochim. et Biophys. Acta 925:258-264. One may use intact cells suspended in a convenient buffer medium containing ascorbate, incubating the cell suspension and then following the reduction of the ascorbate as described above. See, also, Alcain et al. (1991) Biochim. et Biophys. Acta 1073:380-385 and Navas et al. (1992) FEBS Letters 299:223-226.

In assaying for NADH oxidase activity one may measure the rate of disappearance of NADH, the appearance of NAD, or the rate of appearance or disappearance of a reaction product or reactant, respectively, either directly or indirectly. The particular choice of detection will depend on the degree of sensitivity desired, the nature of the sample and the ease of measurement of the signal obtained.

Monitoring of the NADH oxidase can be employed in a number of different situations, for example, to determine whether there is a diseased state, particularly neoplastic state, for monitoring chemotherapy and its effects on tumor growth, for monitoring cancer recurrence after surgery or chemotherapy, and the like. It may also be used in culture, where one is studying agents that may be tumorigenic, monitoring the efficiency of transfection with viruses, studying mutagenesis of cell populations, or the like. The subject system offers numerous opportunities where abnormal cells provide for an NADH oxidase that may be distinguished immunologically or biologically from NADH oxidase from normal cells.

The differences between the NADH oxidase from abnormal cells as compared to normal cells also offers opportunities to screen various agents and their effect on the NADH oxidase associated with the abnormal cells. Since the NADH oxidase appears to be associated with growth of the cells, inhibiting the NADH oxidase activity serves as a cytostatic agent, where the cells prevented from growing will ultimately die. Using a wide variety of agents that are NADH oxidase inhibitors, are physiologically acceptable, and may be used in low concentrations, where the activity may be distinguished between normal cells and abnormal cells, neoplastic cells are treated and their growth inhibited.

NADH oxidase inhibitors of interest include lipophilic fatty acid amides of catecholmethylamines. Of particular interest are fatty acid amides of vanillylmethylamines, where the fatty acids are aliphatic of at least 6 carbon atoms, and may be aliphatically saturated or unsaturated. The compounds may be considered as fatty acid amides of physiologically acceptable aliphatic carboxylic acids and a catecholmethylamine, more particularly, where one of the hydroxy groups is alkylated with an alkyl group of from 1 to 3, preferably 1 carbon atom, i.e. methyl, and the substitution on the ring is 1-aminomethyl-3,4-dihydroxy, where particularly the 3-hydroxy is alkylated. The compositions are used to prevent growth of neoplastic cells in a cell population *in vivo* or in culture, by contacting the cells with neoplastic cell derived NADH oxidase inhibiting amount of one or a combination of the subject compounds.

The fatty acids that acylate the amine will generally be of at least 6 carbon atoms, more usually of at least about 8 carbon atoms, and not more than about 26 carbon atoms, usually not more than about 24 carbon atoms, preferably from about 8 to 18 carbon atoms, more preferably from about 8 to 11 carbon atoms. The aliphatic carboxylic acids may be straight or branched chain, particularly branched at the penultimate carbon atom (Ω-1) (isoalkanoic acids or unsaturated analogs thereof), where the branch will usually be of from 1 to 2 carbon atoms, more usually of 1 carbon atom. Unsaturation may be ethylenic or acetylenic (double or triple bond) where the unsaturation may be at any site in the chain, particularly Ω-4 - Ω-2, where Ω-4 intends unsaturation between Ω-4 and Ω-3, that is between the third and fourth carbon atoms counting from the last carbon atom as one. Fatty acids which may find use include 9-methyldec-7-enoic acid, 9-methyloct-6-enoic acid, lauric acid, caproic acid, oleic acid, stearic acid, palmitic acid, myristic acid, octanoic acid, palmitoleic acids, linoleic acid, arachidonic acid, and the acids naturally found with the vanillylmethylamine as the active component of chili peppers.

The fatty acid amides of vanillylmethylamines will be used in at least partially purified form, usually comprising at least 20 weight %, more usually at least about 50 weight %, conventionally at least about 90 weight % and up to 100 weight % of the subject compounds in a composition for formulation or addition to a culture or other medium. The compounds may be modified by providing for a functional group on the fatty acid, particularly the terminal carbon atom of the fatty acid. Since the compositions may be readily prepared by acylating the catecholmethylamine, any acylating group may be used which has the appropriate biological and chemical activity. The compositions may be used prophylactically, where due to environmental stresses, work, genetic predisposition, accidents or other transforming stimuli, a mammalian host may have an increased propensity for tumor formation. By providing a prophylactic regimen of the subject compounds, the probability of forming cancers dependent on the tumor form of NADH oxidase may be substantially diminished.

Other NADH oxidase inhibitors of interest include anthracyclines, *e.g*. adriamycin and adriamycin conjugates; acetogenins, *e.g*. bullatacin; quassanoids, e.g. simalikalactone D and (-)-glaucarubolone; sulfonylureas, *e.g*. N-(5-indanylsulfonyl)-N'-(4-chlorophenyl) urea (LY186641), N-(4-methylphenylsulfonyl)-N'-(4-chlorophenyl) urea (LY181984) and 4'-aminophenylsulfonyl-4'-(4-chlorophenyl) (LY237868); and chloroaniline.

The active inhibitor may be conjugated to a carrier molecule to enhance the specificity of the subject compounds for neoplastic cells. Of particular interest is conjugation to an impermeant drug or carrier. The use of impermeant drugs or drugs conjugated to an impermeant support allows the active agent to be targeted to the extracellular NADH oxidase, providing additional selectivity and specificity for the drug action. In conjugating the inhibitor to a drug or carrier, a variety of linkers, as known in the art, may be used.

Conveniently, an amino group on the inhibitor may be used for conjugation, or an aminoalkyl derivative of the inhibitor may be used. A large number of heterofunctional compounds are available for linking to entities. Illustrative entities include: azidobenzoyl hydrazide, N-[4-(p-azidosalicylamino)butyl]-3'-[2'-pyridyldithio]propionamide), bis-sulfosuccinimidyl suberate, dimethyladipimidate, disuccinimidyltartrate, N-γ-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, succinimidyl 4-[N-maleimidomethyl]cyclohexane-1-carboxylate and 1-isobutoxy carbonyl-2-isobutoxy-1,2 dihydroquinoline.

Impermeant compounds suitable for conjugation include antibodies directed to molecules on the surface of the targeted cells, *e.g*. tumor cell antigens, virus proteins for targeting to virus infected cells, and the like. Preferred antigens for virus infected cells are the envelope proteins of the virus, particularly HIV-1 gp160 and other retroviral env proteins. Specific antibodies may be coupled to the inhibitors through side chain amino or sufhydryl groups and heterofunctional cross-linking reagents.

Other conjugates that provide targeting to a second cell surface target include ligands for cell surface receptors and proteins, e.g. diferric transferrin for diferric transferrin receptors, growth factors or hormones that have enhanced specificity for one or more cell or organ types, lectins for binding to glycoproteins, *etc*. For example, with estrogen dependent breast cancers, one could couple the subject compound to an estrogen. For T cell leukemias, one would link the subject compounds to an appropriate cytokine, a major histocompatibility antigen which restricts the T cell, or the like.

The impermeant compound may also be a physiologically inactive carrier, including proteins, *e.g*. albumin; polysaccharides, *e.g*. dextran, cyclodextrin, *etc*. or other carrier molecules as known in the art. The polysaccharides may be conveniently oxidized using periodate to provide aldehyde functional groups which can be conjugated to amino substituents, particularly under the conditions of reductive amination. Of particular interest are carriers that have low antigenicity when injected into the host. Ratios of the inhibitor to the carrier may vary widely, usually being at least about 1:1.

In some cases, both partners in the conjugate will be inactive as inhibitors of NADH oxidase, but the conjugate will be active. Conjugates of this type include chloroaniline and quinone site inhibitors that have a low activity as a free molecule, but greatly increased activity when conjugated to an impermeant carrier as described above.

Various techniques may be employed for conjugating an inhibitor of NADH oxidase with a ligand or carrier. A large number of different active agents may be employed where the carboxyl of one member of the conjugate may be activated to react with an amino group of the other member of the conjugate. Alternatively, thiol groups may be linked to activated olefins, e.g. maleimide, to form thioethers. Sugars may be cleaved to provide for aldehydes which may be covalently linked to amino groups under reductive amination conditions. The particular manner of linking will vary widely, depending upon the nature of the two members of the conjugate.

Of particular interest is a conjugate of a monoclonal antibody and a plasma membrane NADH oxidase inhibitor. The inhibitor will generally have an ED₅₀ of less than about 20 µM, usually less than about 10 µM, preferably less thana bout 100 nM. The monoclonal antibody and inhibitor may be covalently conjugated by any convenient means, numerous compounds having been conjugated to antibodies, for example, through a cleaved sugar and reductive amination, maleimide formation and reaction with a thiol to form a thioether, with Ellman's reagent and formation of a thioether or disulfide, and the like.

Various agents may be joined to the selective compound to further enhance the therapy. Thus, a wide variety of drugs which have cytotoxic or antiviral activity may be employed, including immunotoxins, perforins, proliferation inhibitors, and the like.

The subject compositions may be used for treating any vertebrate host that may have or is subject to the transformation of cells resulting in neoplasia. The subject compositions may be used with mammals, *e*.*g*. equines, bovines, canines, felines, rodents, *etc.,* particularly primates, more particularly humans.

The subject compositions may be used with any tumorous condition that expresses the aberrant NADH oxidase, including carcinomas, melanomas, sarcomas, leukemias, lymphomas, *etc*., where the tissues involved may be the prostate, mammary, neuronal, testes, lung, cutaneous tissue, lymph node, mucosal tissue, muscle tissue, lymphocytes, ovary, glandular, *e*.*g*. pancreas, and the like. The subject compositions may be used with hyperproliferative tissue, *e*.*g*. precancerous but neoplastic lesions, a single tumor, or metastatic tumors.

An effective dosage of the subject NADH oxidase inhibitors will be administered in accordance with the need of the particular patient. When using the subject compounds for therapeutic purposes, where the compounds are known and their activity *in vivo* has been already demonstrated, one may empirically determine the method of administration, frequency of administration and dosage, or frequently be able to use the dosage conventionally used with the drug, or a lower dosage that will be determined empirically. The dosage may vary depending upon the mode of administration, the nature of the indication, the purpose for which the drug is administrated, and the like. Because of the low toxicity of capsainoids to normal cells, such compositions may be repeatedly administered over various intervals, where usually the accumulated dosage per treatment cycle will be in the range of about 1 mg to 100 mg/kg of body weight, depending upon the efficacy of the particular formulation, the site of administration, the manner of administration, the tumor of origin and the disease stage. Treatment cycles will be repeated as necessary.

The subject drugs may be administered singly or in combination with other NADH oxidase inhibitors. The subject drugs will be administered in a physiologically acceptable vehicle, such as saline, PBS, water, aqueous ethanol, vegetable oil, glycerol, 25% dimethyl sulfoxide, etc. The concentration of the subject compositions will generally be in the range of about 0.003 to 1 % for topically applied creams and salves or ointments, and 1 to 100 mg/ml for other routes, where the amount will vary with the frequency of administration, the nature of the administration, the nature of the tumor, and the like.

Administration may be by any convenient means, such as parenterally, orally, by inhalation, transdermal or the like. Methods of administration may include intravascular, intraperitoneally, subcutaneous, intramuscular, or the like. Administration may be by solution, dispersion, tablet, aerosol, or the like, where the subject compounds are dispersed in a physiologically acceptable medium at the appropriate dosage. The frequency of administration may be with intervals of 4 hours, 1 day, 3 days, 1 week, 1 month or more, depending upon the need of the patient. The particular regimen will be determined empirically, in accordance with the indication, the response to the therapy, and the like.

To further enhance the therapy, one may use consecutively or concurrently with the subject compounds oxidants and reductants, including, but not limited to ascorbic acid, dehydroascorbic acid, glutathione, dithiothreitol, N-acetyl cysteine, cysteine, *etc*. These compounds are used in their normal therapeutic range, or their dosage will be determined empirically in accordance with known procedures. In view of their safe nature, a wide dynamic range is permissible.

In many instances, it may be desirable to use combination therapies, where the subject compositions may be used in conjunction with other known drugs. Drugs which may be used in combination include conventional drugs, such as alkylating agents, e.g. carmustine, antimetabolites, e.g 5-fluorouracil, antibiotics, e.g. adriamycin, bleomycin, and daunomycin, alkaloids, e.g. vinblastine, biological modifiers, e.g. taxol, biological response modifiers, e.g. interleukins, and other agents, e.g. cis-platinum, and the like.

In many cell populations, one may wish to grow normal cells in the substantial absence of neoplastic or other aberrant cells. Where the aberrant cells express the NADH oxidase associated with neoplastic cells, a growth inhibiting amount of the subject compositions may be used in the growth medium. The concentration of the subject compositions in the medium will usually be at least about 10 nM and not more than about 1 µM. In this way cell populations may be expanded or maintained in culture for various-time periods, while preventing the growth of neoplastic cells.

The use of the subject NADH oxidase inhibitors may find application with transplants, *e.g*. bone marrow, organs, blood, and the like, that are maintained in culture prior to being administered to a recipient mammalian host. In addition, for research purposes, where cells are established in culture for use in studying cellular growth and proliferation, changes in the metabolic response to various compounds or stimuli, maintenance at a primitive level, or the like, overgrowth with neoplastic cells can be prevented by use of the subject NADH oxidase inhibitors in the media.

The subject NADH oxidase inhibitors may also be used to direct various compositions to neoplastic cells. By using the subject compounds as markers on liposomes, where cytotoxic drugs are included in the lumen of the liposome, the liposomes may be preferentially directed to neoplastic cells. Capsaicinoids may be provided with the acyl group of at least about 12 carbon atoms, so as to be included in the liposome membrane, or the terminal carbon atom of the acyl group may be functionalized with an hydroxyl or amino group, in accordance with known ways and the functional group acylated with a fatty acid of at least about 12 carbon atoms, particularly 14 to 30 carbon atoms. Liposomes may be made in accordance with conventional ways, using the subject compositions as one of the lipids. By combining cholesterol, phosphatidyl choline, acyl glycerols, and the like with an aqueous solution of one or more drugs of interest and vigorously agitating the mixture, liposomes may be formed, where the liposome membrane will comprise the subject compositions, which will preferentially bind to neoplastic cell associated NADH oxidase.

The subject NADH oxidase inihibitors may also be used in competitive immunoassays, where an inhibitor is conjugated to a haptenic analyte of interest. The conjugation may be carried out in accordance with known ways, where the acyl group is functionalized as described above. The conjugate may compete with analyte in an assay medium comprising the neoplastic cell associated NADH oxidase for antibody to the haptenic analyte. The amount of antibody which binds to the conjugate will be proportional to the amount of hapten in the assay medium. The conjugate will inhibit the NADH oxidase enzyme when unbound and be prevented from inhibiting the enzyme when bound to antibody. Therefore, the enzyme activity can be related to the amount of haptenic analyte in the assay medium. *See*, particularly U.S. Patent No. 3,935,074 .

Inhibitors of NADH oxidase may be employed to inhibit multiple drug resistance of cells. By employing a conjugate of an NADH oxidase inhibitor with transferrin or other stimulator of NADH oxidase from normal cells, one may provide for the inhibition of multiple drug resistance in a variety of diseased cells, where there is an interest in affecting the growth of the cells. Of particular interest is the combination with cytotoxic agents, as in the case of tumors or other diseases where multiple drug resistance may be a factor. Cytotoxic drugs include cisplatin, vinca alkaloids, methotrexate, 5-fluorouracil, *etc.* One can kill cells by restricting a drug to a cell-surface target, with cells which are resistant to a wide variety of drugs that act internally.

NADH oxidase complex purified from plasma membranes of rat liver and resolved on native gels or purified by HPLC consists of a complex of at least three peptide chains with molecular weights of about 34, 52 and 72 kDa. These compounds can be purified to homogeneity by conventional ways. For example, the protein may be solubilized with a non-ionic detergent, Triton X-100, fractionated on an affinity column, e.g. concanavalin A-Sepharose, followed by gel filtration and anion exchange chromatography.

While these three peptides bear some similarities in molecular weight to the mitochondrial NADH-ubiquinone reductase and a bacterial NAD-reducing dehydrogenase which contain 24 and 51 kDa and 24 and 74 kDa subunits, respectively (Pilkington et al., Biochemistry 30:2166 (1991)), there is little or no sequence similarity of any of the peptides of the plasma membrane NADH oxidase with any of the subunits of either the mitochondrial or the bacterial enzymes.

The 34(36) kDa peptide has sequence similarity to known quinone binding proteins indicating the presence of a quinone binding site in the complex. Peptides from the 34(36) kDa peptide from both rat liver and soybean also show some similarity to a thiol oxidoreductase consistent with a key involvement of active thiol groups as evidenced by the oxidase response to N-ethyl maleimide and sulfhydryl agents, such as dithiothreitol and mercaptoethanol.

The material included within the 55 kDa band, which includes the 52 kDa peptide contains at least one protein having sequence homology with a dihydrolipoamide dehydrogenase.

The 72 kDa peptide from the NADH oxidase complex has no identifiable primary sequence homology with any protein in currently available data bases.

There appears to be no association of heme iron in the oxidase complex at any stage of purification. Non-heme iron is prevalent in the plasma membrane. The oxidase activity is inhibited by iron chelators and by analogy with the quinone dehydrogenase of mitochondria. Based on this observation, the three peptides may include one or more iron-sulfur centers in addition to quinone sites.

Inhibitor targets may vary from the portion of a subunit which binds a cofactor to the active catalytic site which binds NADH and transfers the electron to the electron acceptor. Therefore the inhibitors may vary as to structure in relation to the particular binding site target. Inhibitors having quinone-like structure will bind to the quinone-binding subunit.

As reported by Brightman et al. (1992) Biochem. Biophys. Acta 1105:109-117, NADH oxidase from normal rat liver plasma membrane vesicles is stimulated by growth factors and hormones.

| Addition | Concentration | NADH oxidase nmoles/min/mg protein |
|---|---|---|
| None | | 0.72 ± 0.06 |
| Diferric transferrin | 3 µM | 1.35 ± 0.04 |
| Epidermal growth factor | 27 nM | 1.08 ± 0.03 |
| Insulin | 0.5 +ng/ml | 1.20 ± 0.11 |
| Pituitary extract | 2.5 µl/ml | 1.48 ± 0.15 |
| Retinoic acid | 0.1 µM | 0.54 ± 0.08 |
| Calcitrol | 0.1 µM | 0.16 ± 0.04 |

With hepatoma plasma membranes, the oxidase is elevated and no longer subject to stimulation by added growth factors. The activity of the hormone- and growth-stimulated NADH oxidase responds to guanine nucleotides. The NADH oxidase is activated by mastoparan, but aluminum fluoride is weakly inhibitory. Cholera and pertussis toxins elicit only marginal responses. The activity is inhibited in the presence of Mg²⁺ by GDP and GTP [γ-S], indicating guanine nucleotide regulation of the plasma membrane NADH oxidase.

As is characteristic of many ectoproteins of the mammalian cell surface, an NADH oxidase activity is present in the serum and in HeLa cell culture filtrate. The NADH oxidase activity of culture medium with HeLa cells is proportional to time of incubation and to the amount of medium added. The response to NADH concentration is biphasic with a Kₘ at low NADH concentrations of 15 µM. The activity is inhibited by 85°C for 20 min, trypsin, N-ethyl maleimide and PCMB, and guanosine nucleotides. These activities have properties similar to those of the plasma membrane-bound form. The circulating form appears to have a lower molecular weight (ca. 32 kD) than the membrane-bound form. The circulating NADH oxidase in the serum of cancer patients is inhibited by adriamycin whereas the circulating NADH oxidase in the serum of healthy individuals is not. Further details from this observation are found in the examples.

The following examples are offered by way of illustration and not by way of limitation.

### EXPERIMENTAL

### Example 1

### Methods

*Growth of cells.* HeLa cells (ATCC CCL2), were grown in 150 cm² flasks in Minimal Essential Medium (Gibco), pH 7.4. at 37°C with 10% bovine calf serum (heat-inactivated), plus 50 mg/l gentamycin sulfate (Sigma). Cells were trypsinized with Sigma IX trypsin for 1 to 2 min, harvested by scraping and taken up in TD-tris buffer (140 mM NaCl, 5 mM KCl, 0.7 mM Na₂HPO₄ and 25 mM Tris, pH 7.4) to a final cell concentration of 0.1 g wet weight (gww) per ml.

*Purification of plasma membranes from rat liver.* A 5000 x g pellet from the preparation of Golgi apparatus (Morré (1971) Meth. Enzymol. 22, 130-148) was the starting material. The fluffy layer which contains the Golgi apparatus fraction was mixed, withdrawn and excluded from the plasma membrane preparations. Cold 1 mM NaHCO₃ (5 ml) was added to each tube and the friable yellow-brown upper part of the pellet was resuspended with a pen-brush, leaving the reddish tightly packed bottom part of the pellet undisturbed. The resuspended material was homogenized in aliquots of 5 ml each in a 30 ml stainless steel homogenizer (Duragrind) 20 times by hand. The homogenates were combined, diluted with cold 1 mM NaHCO₃ (1:1 dilution), and centrifuged at 6000 x g in a HB rotor for 15 min. The supernatant was discarded and the pellet was used for the two-phase separation (Morré & Morré (1989) BioTechniques 7:946-958).

The two-phase system contained 6.4% (w/w) Dextran T-500 (Pharmacia), 6.4% (w/w) polyethylene glycol) 3350 (Fisher), and 5 mM potassium phosphate buffer (pH 7.2) (Morré & Morré, 1989, *supra*)*.* The homogenate (1 g) was added to the two-phase system and the weight of the system was brought to 8 g with distilled water. The tubes were inverted vigorously for 40 times in the cold (4°C). The phases were separated by centrifugation at 750 rpm (150 x g) in a Sorvall HB 4 rotor for 5 min. The upper phases were carefully withdrawn with a pasteur pipette, divided in half and transferred into 40 ml plastic centrifuge tubes and diluted with cold 1 mM NaHCO₃. The purified plasma membranes were collected by centrifugation at 10,000 x g in a HB rotor for 30 min. Plasma membrane pellets were resuspended in 50 mM Tris-Mes buffer (pH 7.2) and stored at -70°C. Proteins were determined using the bicinchoninic acid (BCA) assay (Smith et al. (1985) Anal. Biochem. 100: 76-85) with bovine serum albumin as standard. Yields were approximately 3-5 mg per 10 g liver (Navas et al. (1989) Cancer Res. 49: 2146-2147).

The plasma membrane preparations from rat liver have been characterized extensively based on both morphological and enzymatic criteria (Morré (1971) *supra;* Smith *et al.* (1985) *supra*). From morphometric analysis using electron microscopy, the preparations contain 90 ± 4 percent plasma membrane. Contaminants include mitochondria (4%) and endoplasmic reticulum (3%). Based on analyses of marker enzymes, the contamination by endoplasmic reticulum was estimated to be 3%, that of mitochondria 15% and that of Golgi apparatus 1%. The recovery of plasma membranes was estimated to average 18% based on recovery of enzyme markers.

*Purification of plasma membranes from HeLa cells.* HeLa cells were grown as suspension cultures in minimum essential medium (MEM) supplemented with horse serum, diferric transferrin and heparin. Cells were collected by centrifugation for 6 to 15 min at 1,000 to 3,000 rpm. The cell pellets were resuspended in 0.2 mM EDTA in 1 mM NaHCO₃ in an approximate ratio of 1 ml per 10⁸ cells and incubated on ice for 10 to 30 min to swell the cells. Homogenization was with a Polytron Homogenizer for 30 to 40 sec at 10,500 rpm using a PT-PA 3012/23 or ST-probe in 7 to 8 ml aliquots. To estimate breakage, the cells are monitored by light microscopy before and after homogenization. At least 90% cell breakage without breakage of nuclei was achieved routinely.

The homogenates were centrifuged for 10 min at 1,000 rpm (175 g) to remove unbroken cells and nuclei and the supernatant was centrifuged a second time at 1.4 x 10⁶ g min (e.g. 1 h at 23,500 g) to prepare a plasma membrane enriched microsome fraction. The supernatant was discarded and the pellets were resuspended in 0.2 M potassium phosphate buffer in a ratio of approximately 1 ml per pellet from 5 x 10⁸ cells. The resuspended membranes were then loaded onto the two-phase system constituted on a weight basis as described above for rat liver. The upper phase, enriched in plasma membranes, was diluted 5-fold with 1 mM sodium bicarbonate and the membranes were collected by centrifugation. The purity of the plasma membrane was determined to be > 90% by electron microscope morphometry. The yield was 20 mg plasma membrane protein from 10¹⁰ cells.

*Spectrophometric assay of NADH oxidase.* NADH oxidase activity was determined as the disappearance of NADH measured at 340 nm in a reaction mixture containing 25 mM Tris-MES buffer (pH 7.2), 1 mM KCN to inhibit the low levels of contaminating mitochondrial oxidase activity, and 150 µM NADH at 37°C with constant stirring. Activity was measured using a Hitachi U3210 spectrophotometer with continuous recording over two intervals of 5 min each. A millimolar extinction coefficient of 6.22 was used to determine specific activity.

*Purification of Plasma Membrane NADH oxidase.* An aqueous 2-phase partition preparation of rat liver plasma membranes is combined with 1 mM EDTA, 50 mM Tris pH 7.6, and a 10% glycerol wash for solubilization. The solubilized protein is then concentrated with a DE52 ion exchange column, followed by TSK gel filtration chromatography and hydroxyapatite chromatography. The protein may then be purified by SDS-PAGE. Further purification is ahcieved using Centricon removal of SDS and protein concentration, followed by C-18 reverse phase chromatography.

### Response pattern of the NADH oxidase from the plasma membrane of normal and cancer cells and tissues.

With plasma membranes from rat liver, a normal plasma membrane source, the NADH oxidase activity is resistant to inhibition by the antitumor drug adriamycin with an ED₅₀ of about 7 µM. In contrast, with NADH oxidase activity stimulated by epidermal growth factor (EGF), growth factor-stimulated activity is inhibited completely by 0.1 µM adriamycin.

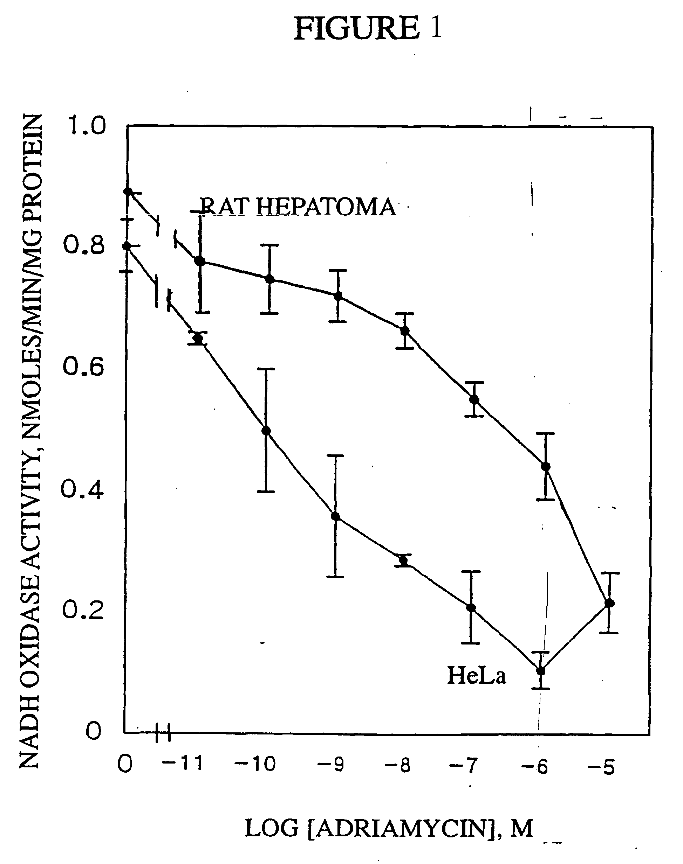

With plasma membranes of rat hepatomas, the NADH oxidase activity is inhibited by adriamycin and is no longer growth factor-responsive, but appears to be constitutively activated. The constitutively activated plasma membrane NADH oxidase of the rat hepatoma is inhibited preferentially by low concentrations of adriamycin, as shown in Figure 1. At 0.1 µM adriamycin, the activity is 40% inhibited with hepatoma plasma membranes, but was uninhibited with liver plasma membranes. As the concentration of adriamycin exceeds 1 µM the NADH oxidase activities of both liver and hepatoma plasma membranes are inhibited. Results with plasma membranes of human ovarian carcinoma cells grown in culture (HeLa) demonstrated an ED₅₀ of adriamycin for inhibition of NADH oxidase of 0.7 nM, as shown by the data in Figure 1.

That the adriamycin inhibits some electron transport activity associated with the plasma membrane is further substantiated by data from an assay which relies on the inhibition of the disappearance of ascorbate radical from 0.2 mM solutions of ascorbate by electrons (reducing equivalents) released from cells (Navas *et al., supra*)*.* Under these conditions, electron flow is inhibited by adriamycin at concentrations where growth is inhibited in adriamycin susceptible HL-60 cells, but not inhibited with adriamycin-resistant cells, where growth also is not inhibited. These inhibitions with susceptible cells were observed without perceptible lag. Also the response was observed without preincubation with adriamycin. These latter sets of observations support the concept that the adriamycin does not enter the cell nucleus to cause inhibition of the transmembrane electron flow via effects on enzymes of membrane formation through inhibition of DNA synthesis.

### Use of Plasma Membrane NADH Oxidase in a Screening Procedure to Identify Anticancer Drugs

Because the NADH oxidase protein is altered in its responsiveness to growth factor, hormones, as well as anticancer agents, and because cells where the NADH oxidase is inhibited do not grow, the NADH oxidase provides an important target for development and design of new and/or improved antitumor agents of a high degree of efficacy and specificity. The ability of the NADH oxidase activities to be inhibited or of the NADH oxidase to bind drugs can be utilized as a rapid *in vitro* screen to predict antitumor efficacy. Comparisons with plasma membranes from normal cells in parallel provide specificity. Substances are sought that, at low concentrations, inhibit completely the NADH oxidase of target cells without affecting the NADH oxidase of non-target cells even at 10- to 100-fold higher concentrations than those required to inhibit the target cell NADH oxidase.

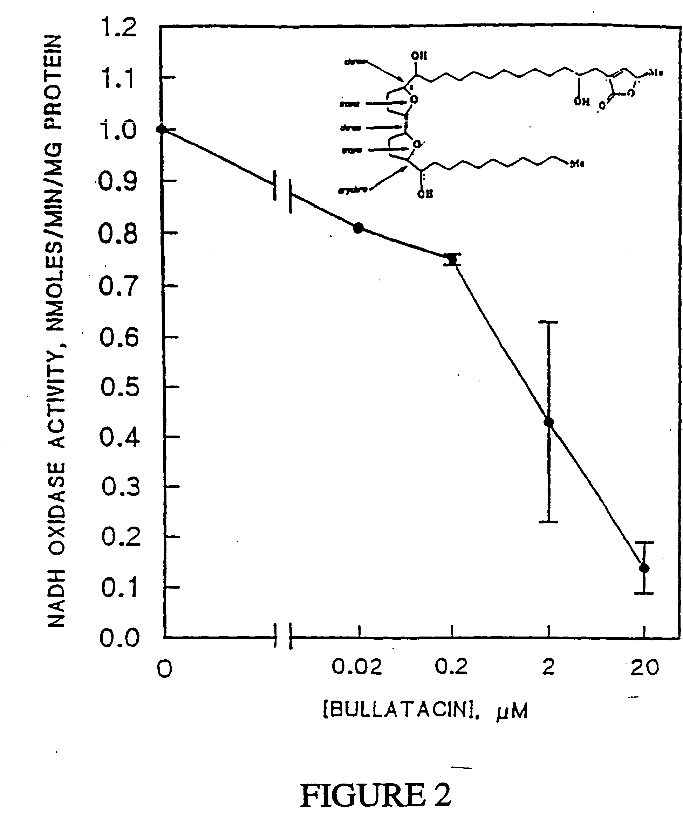

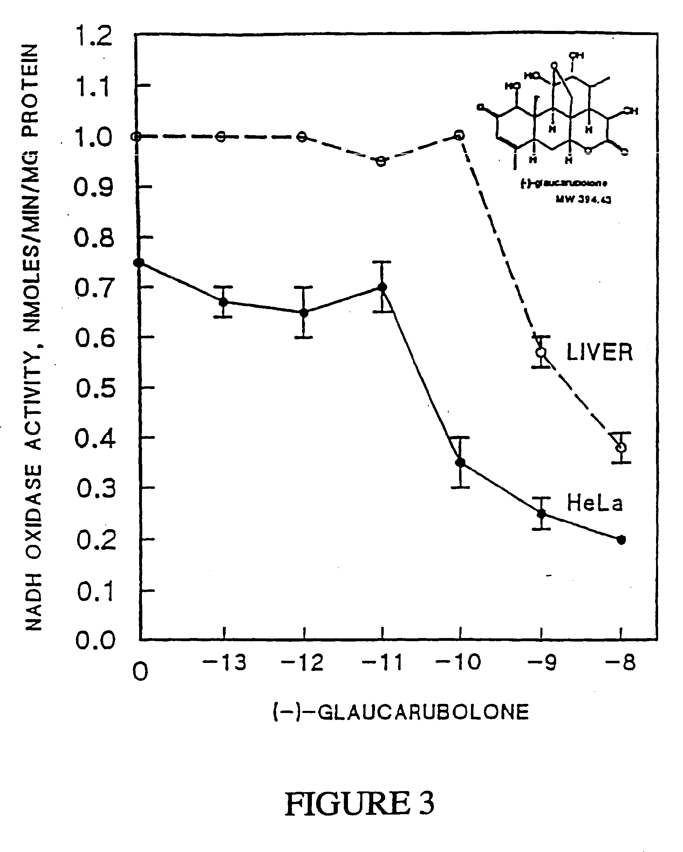

Among the classes of drugs with antineoplastic or antitumor activity where efficacy in inhibiting the NADH oxidase and of growth in parallel are included the following:
a) Adriamycin and adriamycin conjugates. Adriamycin inhibits both the NADH oxidase of rat hepatoma plasma membrane and the NADH oxidase of plasma membrane of HeLa cells at low adriamycin concentrations, as shown in Figure 1, but the NADH oxidase activity of plasma membrane of normal liver is not inhibited below the micromolar range of adriamycin concentrations.
b) Acetogenins. The acetogenins of which bullatacin is an example, inhibit NADH-quinone reductases of mitochondria and exhibit antitumor activity. They inhibit the NADH oxidase as well, illustrated by the data shown in Figure 2. The inhibitions occur over a range of concentrations similar to that causing inhibition of tumor cell growth.
c) Quassanoids. The quassanoids are a further class of compounds with anticancer activity, having an unknown mode of action (Moher et al. (1992) J. Am. Chem. Soc. 114:2264; Gross et al. (1990) ibid 112:9430). Two members of the series, simalikalactone D and (-)-glaucarubolone, have been tested and inhibit the NADH oxidase over ranges of concentrations similar to those that inhibit cancer growth, illustrated by the (-)-glaucarubolone inhibition data shown in Figure 3. The NADH oxidase activity of rat hepatoma plasma membrane is inhibited by the quassanoid, similikalactone D at a final concentration of 0.4 µM prepared in DMSO, where an equivalent amount of DMSO was without effect.
(d) Sulfonylureas. Diarylsulfonylureas, such as N-(5-indanylsulfonyl)-N'-(4-chlorophenyl) urea (LY186641, Sulofenur), N-(4-methylphenylsulfonyl)-N'-(4-chlorophenyl) urea (LY181984) and 4'-aminophenylsulfonyl-4'-(4-chlorophenyl) (LY237868) have been shown to be effective antitumor agents in a variety of *in vivo* and *in vitro* animal models. (Rush et al. (1992) Biochem Pharmacol 44:2387-94). Their mechanism of action is unknown. Mitochondria have been shown to accumulate Sulofenur and therefore may be targets of drug action. Many diarylsulfonylureas that are effective antitumor agents are also uncouplers of mitochondrial oxidative phosphorylation.

### Use of Plasma Membrane NADH Oxidase as A Target for Drugs to Bypass Multiple Drug Resistance

A conjugate of adriamycin with transferrin has been reported to be in the order of 10 times more active in the inhibition of growth cancer cells in culture than unconjugated drug (Faulk et al., Lancet 2:390-392 (1980); Faulk et al., Mol. Biotherm. 2:57-60 (1990); Faulk et al., In Oxidoreduction at the Plasma Membrane: Relation to Growth and Transport (Crane, F.L., Morré, D.J., and Löw, H., eds.) CRC Press, Boca Raton, pp. 205-224; Sun et al., Biochim. et Biophys. Acta 1105:84-88 (1992); and references cited therein). This conjugate also has the interesting property of being able to inhibit the growth of adriamycin-resistant cell lines. This inhibition occurs regardless of the mechanism of resistance (*e.g*., expression of the MDR gene encoding the 170 kDa P-glycoprotein of the plasma membrane). It is not necessary for the adriamycin conjugates to enter the cells to be cytotoxic and neither the conjugates nor free adriamycin derived from the conjugates reach the cell nuclei in concentrations sufficient to be cytotoxic.

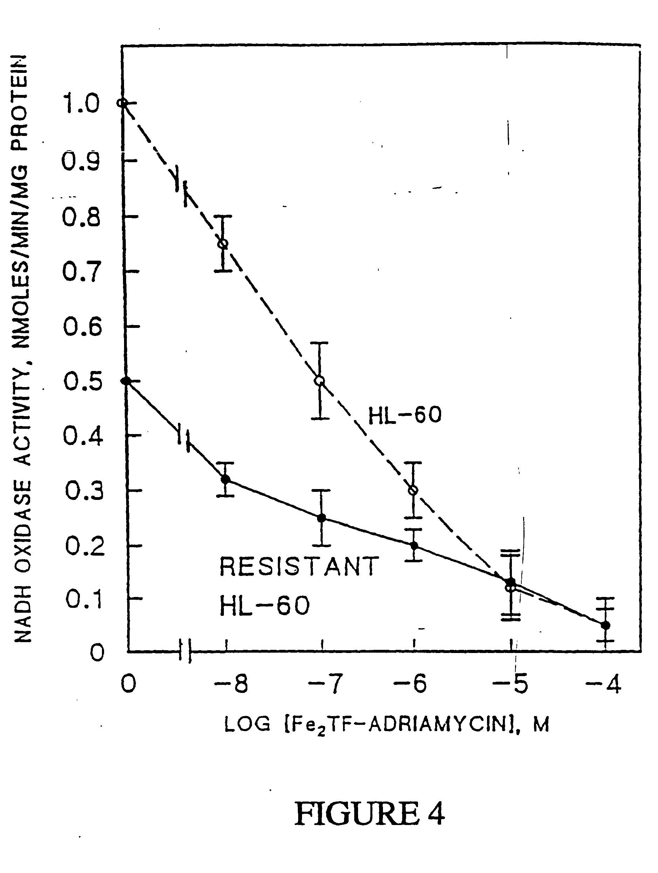

As documented previously, electron transport out of HL-60 cells is inhibited by adriamycin. However, in resistant cells, neither growth nor electron transport are inhibited. Similarly, the NADH oxidase of adriamycin-resistant HL-60 cells is not inhibited by adriamycin whereas that of normal HL-60 cells is inhibited. A conjugate of adriamycin with transferrin (provided by Dr. W. Page Faulk, Center for Reproduction and Transplantation Immunology, Methodist Hospital, Indianapolis) is known to inhibit growth of adriamycin-resistant cells. This conjugate, unlike free adriamycin, inhibits the NADH oxidase activity of plasma membranes of both adriamycin-resistant and adriamycin-susceptible HL-60 cells, illustrated by the data shown in Figure 4. This is in contrast to plasma membranes of liver where the NADH oxidase activity is stimulated rather than inhibited by the conjugate. The activity stimulated by diferric transferrin in rat liver plasma membranes may be a response to the transferrin portion of the conjugate.

In the example provided, multiple drug resistance involving the NADH oxidase is overcome when the drug is modified to inhibit the resistant oxidase. These observations support the exploitation of the plasma membrane NADH oxidase as a target to overcome multiple drug resistance, by combining an NADH oxidase inhibitor with a drug susceptible to multiple drug resistance in a combined therapy, where the two drugs may be administered to the target site simultaneously, as individual compounds, or as a covalent conjugate, as a non-covalent complex in a liposome or other convenient carrier.

### Use of Plasma Membrane NADH Oxidase as a Marker for Cancer Detection and Diagnosis

The subject of this example is the utilization of a circulating form of the NADH oxidase of cancer cells specifically inhibited by adriamycin and other antitumor drugs that act on the cell surface target as the basis for cancer detection. This embodiment of the invention is based upon the observation that the-target is located on the cell surface and subsequently shed from normal and tumor cells into the blood. A characterization of NADH oxidase activity from normal human serum shows that the activity is proportional to time of incubation and to serum concentration. The response to NADH concentration is biphasic with a Kₘ at low NADH concentrations of 25 µM. The activity is inhibited by GTP, 85°C for 20 min, trypsin, N-ethyl maleimide and p-chloromercuribenzoate (PCMB). Additionally, the NADH oxidase activity of serum (or some portion thereof) was more susceptible to inhibition by adriamycin with serum of cancer patients than with serum of normal individuals. This was proven to be correct by experiment both for tumor-bearing rats (Table 1) and for sera of cancer patients (Table 2).

Results from serum of 12 normal individuals and 18 cancer patients are summarized in Table 2. With sera from normal individuals, adriamycin consistently stimulated the change in absorbance. However with the sera from the 18 cancer patients, the activity was inhibited with 17 of the samples and unchanged in one.

Assays of circulating enzymatic activity offer the opportunity for early detection and monitoring of cancer and for isolation of a circulating cancer-specific isoform of the hormone- and growth factor-stimulated NADH oxidase, which can serve as a target for antineoplastic agents. Additionally, the activity provides a non-invasive procedure for prediction of therapeutic response to chemotherapeutic agents for cancer control.

**TABLE 1**

| Relative rates of NADH oxidation by serum samples from normal rats or rats bearing an RLT-28 tumor in response to 0.25 µM adriamycin. | | |
|---|---|---|
| | **NADH oxidation** | |
| **Serum** | **No adriamycin** | **+0.25 µM adriamycin** |
| Normal | 1.7 ± 0.1 | 1.75 ± 0.05 |
| Tumor (RLT-28) bearing | 1.0 ± 0.1 | 0.35 ± 0.05 |

**TABLE 2**

| Inhibition of NADH oxidase activity by adriamycin in human serum samples from cancer patients. | | | |
|---|---|---|---|
| Sera from normal individuals | | Sera from cancer patients | |
| Designation | Δ absorbance | Designation | Δabsorbance |
| Normal 1 | - 0.25 | Rectal carcinoma | + 0.4 |
| Normal 2 | - 0.2 | Colon carcinoma | + 0.35 |
| Normal 3 | - 0.05 | Breast carcinoma | + 0.3 |
| Normal 4 | - 0.06 | Breast carcinoma | + 1.2 |
| Normal 5 | - 0.5 | Ovarian carcinoma | + 0.4 |
| Normal 6 | - 0.37 | Small cell lung carcinoma | + 0.45 |
| Normal 7 | - 0.05 | Colon carcinoma | + 0.3 |
| Normal 8 | - 0.4 | Chronic lymphocytic carcinoma | + 0.0 |
| Normal 9 | - 0.4 | Hairy cell leukemia | + 0.8 |
| Normal 10 | - 0.4 | Osophogeal carcinoma | + 0.6 |
| Normal 11 | - 0.2 | Prostate carcinoma | + 0.4 |
| Normal 12 | - 0.35 | Prostate carcinoma | + 1.2 |
| | | Prostate carcinoma | + 0.3 |
| | | Breast cancer | + 0.6 |
| | | Myeloma | + 1.2 |
| | | Lymphoma | + 1.4 |

| | | | |
|---|---|---|---|
| Negative values indicate stimulation of activity. Positive values are inhibition. Reported are changes in absorbance at 360 nm (Δ absorbance) due to addition of 0.25 µM adriamycin comparing serum samples from normal and cancer patients. Serum was stored frozen, 100 µl samples were analyzed. A control rate was established over 10 min and the rates reported are from the second 5 min after addition of adriamycin. | | | |

As evidenced by the above results, the use of NADH oxidase as a target provides for many opportunities in the diagnosis and treatment of diseased states. Cancer cells may be diagnosed and treated by detecting NADH oxidase specifically associated with neoplastic cells by immunological or chemical means. Alternatively, by targeting NADH oxidase with specific active agents, multiple drug resistance may be inhibited, and neoplastic cell growth may be curtailed.

Using plasma membrane NADH oxidase as a therapeutic target permits the use of impermeant drugs or drugs conjugated to an impermeant support, which act on an extracellular target, avoiding the many difficulaties associated with drugs which act on an intracellular target, such as cytoplasmic or an organelle, where the drug must be able to cross the plasma membrane, survive the cytoplasmic environment, and in many cases cross the organelle membrane. Furthermore, by targeting the NADH oxidase associated with neoplastic cells, compounds may be screened for NADH oxidase inhibition activity, so as to find utility in culture or *in vivo* for preventing the growth of neoplastic cells.

### Example 2

### Characterization of Protein Disulfide Interchange activity and Inhibition by an Antitumor Sulfonylurea

Protein disulfide isomerase or protein disulfide isomerase-like enzymes are multifunctional proteins that catalyze disulfide-thiol interchange reactions in protein substrates leading to net protein disulfide reduction, formation of isomerization, or thiol oxidation depending on the initial substrates and the thiol-disulfide potential imposed by the environment (e.g., the redox buffer). In this example it is demonstrated that a protein disulfide isomerase-like activity using plasma membrane vesicles isolated from HeLa cells is responsive to the antitumor sulfonylureas. The sulfonylurea LY181984, but not the structurally-related, inactive compound N-(4-methylphenylsulfonyl)-N'-(phenyl)urea (LY181985), inhibited the activity. The sulfonylureas, known collectively as antitumor diarylsulfonylureas, are a class of synthetic organic compounds having activity against human tumors *in vivo* (Howbert et al. (1990) J. Med. Chem. 33:2392-2407). Their mechanism of action is unknown, and apparently unrelated to previously described classes of oncolytic agents.

### Methods

Cell growth, purification of plasma membranes and spectrophotometric assays were performed as described in Example 1.

*Estimation of protein disulfide isomerase- (PDI) like activity.* Two methods were employed to determine PDI or PDI-like activity. Both involved restoration of ribonuclease activity by refolding of reduced and randomly oxidized (scrambled) or reduced and inactive ribonuclease A as a measure of the activity.

In the first method, ribonuclease A activity was measured using a spectrophotometric assay based on cCMP as RNAse substrate. Scrambled RNAse (90.363 mg) was incubated together with 0.45 mM cCMP in 50 Mm Tris-MES buffer, pH 6.5, at 30°C in a final volume of 3 ml. In the absence of membranes, the scrambled RNAse became active as evidenced from an increase in A₂₉₆ from the RNAse catalyzed hydrolysis of cCMP. A variation of this assay was to preincubate scrambled RNAse with transitional endoplasmic reticulum for 20 min in the presence or absence of 1 µM all-trans retinol (using ethanol as control) and in the presence or absence of 1 µM reduced or oxidized glutathione or a mixture of both. After this 20 min of "reactivation" of RNAse, cCMP was added to a final concentration of 0.45 mM to initiate the reaction. The increase of the absorbance at 296 nm was recorded over 25 min and the concentrations of cCMP were determined (E = 0.19 mM⁻¹cm⁻¹). Spectrophotometric measurements were with a Hitachi U3210 spectrophotometer with a thermostatic cell compartment maintained at 30°C with continuous stirring.

The second method determined the rate of hydrolysis of yeast RNA by measuring the amount of acid-soluble oligonucleotides liberated. 250 µl of 0/15 sodium acetate buffer, pH 7.0, 550 µl of reagent grade water, 10 µl of 0.1 mg/ml retinol, 10 µl of transitional endoplasmic reticulum (400 µg protein) and 50 µl of scrambled or reduced RNAse (360 mg/ml) were preincubated in a 1.5 ml Eppendorf centrifuge tube for 20 min at 37°C. For the assay, 100 µl was transferred to each of 4 tubes. Yeast RNA (100 µl, 0.33 mg/ml, pH 5.0) was added. After 0 min and 10 min, the reaction was stopped by the addition of 100 µl of 25% percholoric acid containing 0.75% uranyl acetate. The tubes were cooled in an ice bath for 5 min and clarified by centrifugation (2 min, microfuge). The 75 µl of the clear supernatant was diluted to 3.0 ml with water. The absorbance was measured at 360 nm against an unincubated blank. One unit is defined as an increase in absorbance of 1.0 at A₂₆₀ at 37°C and pH 5.0.

Protein was estimated by the method of Smith et al. (1985) Annal. Biochem. 100:76-85.

*Preparation of scrambled RNAse substrate.* To prepare the oxidized, denatured RNAse substrate (scrambled RNAse), native RNAse A (Sigma, Type 1-AS from bovine pancreas) (30 µg/ml) was incubated 1 h at 35°C in 50 mM Tris-acetate, pH 8.6, containing 9 M urea and 130 mM DTT.

The fully reduced protein was isolated by adjusting the pH to 4.0 with glacial acetic acid followed by elution from a column of Sephadex G-25 with degassed 0.1 M acetic acid and used directly or further processed to produce scrambled RNAse. Protein concentration was estimated from spectrophotometric measurement at 280 nm using native RNAse A as standard. For preparation of scrambled RNAse, the reduced RNAse was diluted to 0.5 mg/ml with 0.1 M acetic acid. Solid urea was then added to a final concentration of 10 M after which 0.1 M sarcosine hydrochloride was added and the pH adjusted to 8.5 with 1 M Tris. The mixture then was incubated in the dark for 2-3 days during which time the protein was randomly oxidized. The scrambled product was recovered by acidification to pH 4 with glacial acetic acid and elution from Sephadex G-25 in 0.1 M acetic acid. Fractions containing protein were pooled, adjusted to pH 8 and stored at 4°C. Free thiol groups, as determined using 5,5' dithio(2-nitrobenzoic acid), were 80 to 90% oxidized.

### Results

When RNAse was assayed by the method of Kalnitsky et al. (1959) J. Biol. Chem. 234:1512-1519, HeLa cells exhibited a weak protein disulfide isomerase-like activity as evidenced by the restoration of activity to scrambled RNAse, data shown in Table 3. In the presence of reduced glutathione (GSH), this weak activity was stimulated approximately 2- to 3-fold by the addition of NADH. When RNAse activity was estimated using a cCMP as substrate, a weak activity also was observed but only when preincubated in the presence of GSH, shown in Table 4.

**Table 3**

| Effect of sulfonylurea and thiol compounds on protein disulfide-thiol interchange of HeLa plasma membranes | | | | |
|---|---|---|---|---|
| Addition, 1 µM | Sulfonylurea | | 0.D./10 min | |
| | | I | II | III |
| None | None | 0.11 | 0.047 | 0.021 |
| | LY181984 | 0.076 | 0.049 | - |
| GSH | None | 0.06 | 0.06 | 0.041 |
| | LY181984 | 0.056 | 0.03 | 0.018 |
| GSSG | None | 0.093 | 0.09 | 0.026 |
| | LY181984 | 0.044 | 0.04 | 0.016 |

**Table 4**

| Protein disulfide-thiol interchange activity of HeLa plasma membrane | |
|---|---|
| Treatment | nmoles/min/mg protein |
| None | 1.6 |
| 1 µM LY1984 | 1.0 |
| 1 µM capsaicin | .95 |
| GSH | 2.1 |
| GSSG | 1.1 |

The sulfonylurea was prepared and added in DMSO. The concentration dependency of the sulfonylurea inhibition exhibited a broad optimum in the presence of GSH for activation of both reduced and scrambled RNAse. GSSG also promoted activation. DMSO controls were evaluated and the solvent, on average, was without effect. When measured in the presence of NADH, NADH stimulated the protein disulfide-like isomerase activity. Again, the sulfonylurea was inhibitory.

HeLa plasma membranes also exhibit an ability to oxidize NADH in the presence of potassium cyanide to inhibit mitochondrial activity, where the cyanide-resistant NADH oxidase activity is inhibited by sulfonylurea.

The sulfonylurea had no effect on activated RNAse alone, as shown in Table 5.

**Table 5**

| Effect of sulfonylureas on activity of native RNAse at pH 6.0 and pH 6.8 | | |
|---|---|---|
| Sulfonylurea, 1 µM | µmol/mg/min | |
| | pH 6.0 | pH 6.8 |
| None | 0.15 | 0.21 |
| LY181984 | 0.14 | 0.21 |
| LY181985 | 0.15 | 0.21 |

Two different methods were used to assay protein disulfide isomerase-like or protein disulfide-thiol interchange activity of HeLa plasma membranes and the response of the activity to sulfonylureas. The active antitumor sulfonylurea LY181984 inhibited the refolding of RNAse depending on the redox buffer and conditions of preincubation. With no preincubation, or with membranes preincubated with GSH or a mixture of GSH and GSSG, LY181984 inhibited the activation of scrambled RNAse. The findings suggest that the response is dependent both on the redox state of the initial substrate and on the imposed thiol-disulfide potential as generated by the surrounding redox buffer. If the redox state is unbalanced, either with GSH or GSSG alone, sulfonylurea stimulations were still observed. The activity inhibited by sulfonylurea appears to be distinct from the classic protein disulfide isomerase of the endoplasmic reticulum lumen, and also distinct from an oxido-reductase activity obligatorily driven by pyridine nucleotide substrates. The data suggests that the protein responsible for plasma membrane NADH oxidase activity also has protein disulfide-thiol interchange activity.

### Example 3

### Inhibition of Plasma Membrane NADH Oxidase with Capsaicin

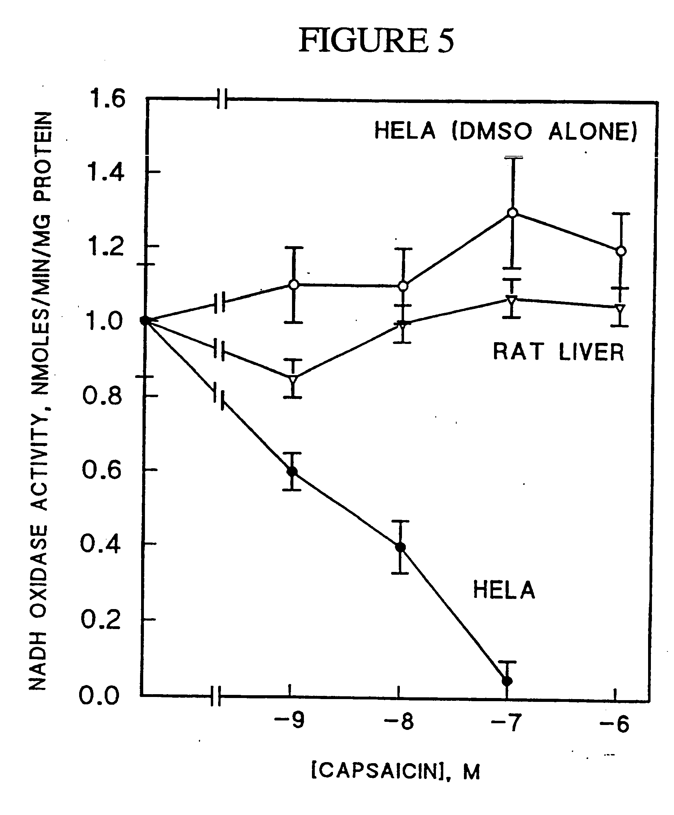

Cells were grown, and plasma membranes prepared, as described in Example 1. The dose response of NADH oxidase activity from plasma membranes from rat liver to capsaicin was determined. As shown by the data presented in Figure 5, capsaicin had no effect on the NADH oxidase activity from the plasma membrane of normal cells. However, with plasma membrane from HeLa cells, the activity was inhibited by capsaicin, with an ED₅₀ of about 50 nM. At 0.1 µM capsaicin, the activity was nearly completely inhibited.

In addition to inhibition of NADH oxidase of plasma membranes, capsaicin also inhibited the growth of attached HeLa cells in culture. Inhibition of growth of attached HeLa cells during 72 h of culture by capsaicin under normal conditions of cell culture was observed. The number of cells initially plated was subtracted. The results were averages from duplicate experiments. Growth was 50% inhibited at about 1 µM. However, with attached HeLa cells where the growth factor conditions were manipulated to increase drug efficacy, growth was inhibited completely at a concentration of capsaicin of 10 to 100 nM. This result correlated closely with the inhibition of NADH oxidase activity of the HeLa cell plasma membranes.

HL-60 cells grown in suspension were inhibited as well by capsaicin. However if the cells were induced to differentiate with DMSO, the cells became resistant to the inhibitory effects of capsaicin. If after several days following the DMSO-induced differentiation, the cells resumed rapid growth, the capsaicin once again inhibited the growth.

With the HL-60 cells, the NADH oxidase activity of isolated plasma membranes was inhibited as well by capsaicin. However, with plasma membranes isolated from cells freshly induced with DMSO, the NADH oxidase activity appeared to resist inhibition by capsaicin.

As an example of a non-cancer cell type, the growth and NADH oxidase activity of normal rat kidney cells were examined. Neither growth nor NADH oxidase activity of isolated plasma membranes compared to DMSO alone was inhibited by capsaicin except at very high concentrations (> 10⁻⁵ M).

The above results support the conclusion that NADH oxidase activity of HeLa cells (human ovarian carcinoma derivation) are inhibited by capsaicin. In contrast to the observation with HeLa plasma membranes, plasma membranes of normal cells from rat liver or rat kidney were not inhibited. Similarly, the NADH oxidase of HL-60 (human leukemia cells) was inhibited by capsaicin, but upon induction or differentiation with DMSO (Collin et al. (1979) J. Exp. Medicine 14: 969-974), the NADH oxidase activity of the isolated plasma membrane vesicles was much less inhibited.

Not only does capsaicin inhibit the NADH oxidase of plasma membrane vesicles from cancer cells and not from normal cells, the substance exerts a parallel response on growth. Growth is nearly completely inhibited by capsaicin with HeLa and HL-60 cells where rat kidney and HL-60 cells induced to differentiate with DMSO are substantially less affected by the capsaicin.

The effect of capsaicin, which is representative of a group of pungent principals from chile peppers, may be correlated with the observation that there is an inverse ratio between cancer death rates and diets traditionally high in chile peppers. Compared to the United States, cancer death rates at all sites were 157 for males and 106 for females compared to 56 and 71 for Mexico and 29 and 20 for Thailand (the lowest of any country). The capsaicin need not be pure to be active. A shown by the data in Table 6, extracts derived from jalapeña peppers at dilutions of 1:100 and 1:1000 inhibit activity of NADH oxidase of plasma membrane vesicles prepared from HeLa cells.

**Table 6.**

| Response of NADH oxidase activity of HeLa plasma membrane vesicles to an extract (Mashate prepared with no additions) from fresh pericarp of jalapeña peppers tested at dilutions of 1:100 and 1:1000. | | |
|---|---|---|
| | **nmoles/min/mg protein** | Δ |
| Control | 0.9 | |
| + Extract 1:1000 | 0.5 | 0.4 |
| Control | 1.0 | |
| + Extract 1:100 | 0.2 | 0.8 |

Comparing sera of 11 normal individuals, capsaicin was without effect at 100 nM. With sera from 12 cancer patients, activity was inhibited with 9 and stimulated with 3. The results are reported in the following table.

**Table 7.**

| NADH oxidase activities of human serum in response to 100 nM capsaicin. | | | |
|---|---|---|---|
| | **None** | **100 nM capsaicin** | **Ratio** |
| SB-69 Bladder | 0.3 | 0.2 | 0.66 |
| SB-11 Renal cell | 0.6 | 0.45 | 0.75 |
| SB- 4 Prostate | 0.45 | 0.2 | 0.44 |
| SB-58 Bladder | 0.9 | 0.45 | 0.5 |
| SB-76 CML | 1.8 | 1.5 | 0.8 |
| SB-74 Small lung cell | 0.8 | 1.0 | 1.25 |
| SB-65 Lymphoma | 0.6 | 0.9 | 1.5 |
| SB-68 Leukemia | 0.65 | 0.75 | 1.15 |
| SB-77 Colon | 0.9 | 0.6 | 0.66 |
| SB-66 Breast | 0.8 | 0.6 | 0.75 |
| SB-65 Lymphoma | 0.25 | 0.15 | 0.6 |
| SB-72 Lung | 0.6 | 0.4 | 0.66 |
| PU-21 | 0.45 | 0.45 | 1.0 |
| PU-10 | 0.45 | 0.45 | 1.0 |
| PU-25 | 0.6 | 0.53 | 0.9 |
| PU-24 | 0.25 | 0.25 | 1.0 |
| PU-21 | 0.2 | 0.2 | 1.0 |
| PU-22 | 0.6 | 0.625 | 1.04 |
| PU-27 | 1.1 | 1.05 | 0.95 |
| PU-31 | 0.475 | 0.45 | 0.95 |
| PU-29 | 0.825 | 0.8 | 0.97 |
| PU-28 | 0.275 | 0.275 | 1.0 |
| PU-30 | 1.1 | 1.1 | 1.0 |
| Mean | 0.575 | 0.56 | 0.97 |

It is evident from the above results that the subject compositions have a preferential effect in inhibiting growth of tumor cells, while having substantially no effect on normal cell growth. The patient may be monitored for response to the use of the subject compounds by determining the response of the NADH oxidase in the bloodstream. In this way, patients may be conveniently screened for determining their response to the subject therapy. The subject compounds are safe, being naturally occurring substances or analogous thereto, can be used at elevated concentrations with few side effects and do not require transport across the membrane for activity. In addition, the subject compositions can be readily administered in a wide variety of ways, while retaining efficacy. By employing the subject therapy, one can achieve reduction of tumor proliferation, so as to reduce the tumor burden, to allow other therapies to be used, which might otherwise be unacceptable, due to adverse side effects. In addition, the subject therapies do not interfere with the host immune system, but allow for cooperation with the proliferation inhibition of the subject compounds and the cytotoxic activity of the immune system.

### Example 4

### Conjugates of Impermeant Compounds and Plasma Membrane NADH Oxidase Inhibitors

### Methods

The growth of cells, preparation of plasma membranes, and spectrophotometric measurements were performed as described in Example 1.

*Conjugation with a-cyclodextrin -* α-Cyclodextrin (α-CD) was linked to LY237868 (4'-aminophenylsulfonyl)-4'-(4-chlorophenyl) urea using the linking agent 1-isobutoxycarbonyl-2-isobutoxy-1,2-dihydroquinoline. For this procedure, α-CD was reacted with succinic anhydride to form succinolyated cyclodextrin, which was purified by extraction of the impurities with a Sep-Pak C-18 cartridge. The succinoylated cyclodextrin was then reacted with an excess of LY237868 to form the product.

*Conjugation of LY237868 with α-cyclodextrin (α-CD) -* α-CD was dried over 105°C overnight and 1.86 g (1.9 mmol) of α-CD was dissolved in a solution containing 50 ml pyridine (dried over NaOH) and 20 ml dimethylformamide (DMF). To this solution, 30 g of succinic anhydride of 50 ml DMF was added. The reaction was at 50°C for 120 h while stirring. The solution became dark brown with time. At the end of reaction, the solution was concentrated to a minimal volume (syrup) under vacuum at 50°C. The dark brown syrup was dissolved in dry methanol and unreacted excess succinic anhydride was removed by filtration. The methanol solution was dried to a syrup under reduced pressure and dissolved in a minimal amount (15 ml) of double distilled water. Persuccinolyated α-CD was concentrated and dried over phosphorous pentoxide under vacuum for 8 h (2.68 f).

Persuccinolyated α-CD (0.4 g) and 3.6 mmol of LY237868 sulfonylurea (0.89) were dissolved in 6 ml dry methanol. To this solution 0.8 g (4 mmol) of 1-isobutoxy carbonyl carbonyl-2-isobutoxy-1,2 dihydroquinoline (IIDQ) was added. Reaction was overnight at room temperature. The sulfonylurea conjugated α-CD was precipitated by adding 20 ml of water, filtered and dried (0.25 g).

*Synthesis of sulfonylurea-acetic acid conjugate -* Acetic acid N-hydroxy succinimide ester (0.037 g) (0.23 mmol) LY237868 (0.045 g) (0.14 mmol) were dissolved in 2 ml dry methanol (dried over molecular sieve). The reaction was for 5 h at room temperature with stirring. The conjugate was purified by silica gel chromatography (50 ml bed volume, Iatrobeads, Iatron Laboratories, Inc., Japan) (MeOH: CH₂Cl₂=10:1). Fractions having the conjugate (R_{f} value = 0.79) were pooled and dried (26 mg, 0.07 mmol).

### RESULTS

### Sulfonylurea α-Cyclodextrin Conjugates

To test the concept that the NADH oxidase inhibited by sulfonylurea was accessible to NADH from the external surface, an impermeant conjugate of an LY181984 analogue substituted with an amino group in position 3 of the B ring (LY237868) was utilized. This amino group was then used to link the sulfonylurea to an impermeant cyclodextrin.

Cross-linking was verified by the disappearance from the infrared spectrum of the free amino function and its replacement by an amide bond. In this study, LY237868 was linked to acetic acid using the same active ester chemistry as used in preparing the cyclodextrin conjugate. In this procedure, acetic acid was converted to the N-hydroxysuccinimide active ester and then linked to the LY237868 to form the 4'-acylamine derivative. The product was conclusively identified by determining its exact mass spectrometry: Found, 367.03767; Calcd., 367.03936 for C₁₅H₁₄N₃O₄SCl.

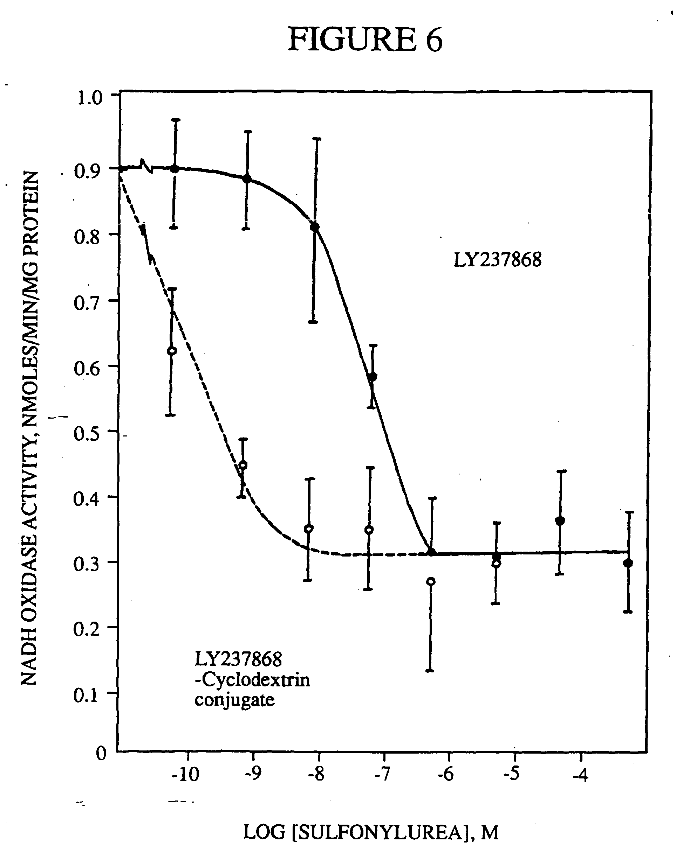

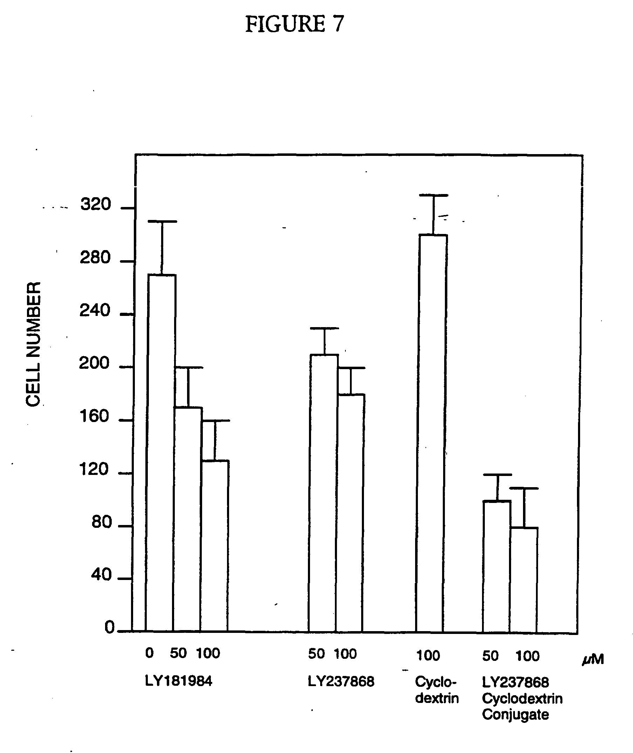

On a sulfonylurea basis, the cross linked compound was as active or even more active than the parent compound in inhibiting the NADH oxidase of HeLa plasma membranes. Inhibition by 50% of the activity was observed at a conjugate concentration equivalent to 1 nM LY237868. The data is shown in Figure 6. When tested for inhibition of growth of HeLa cells, the conjugate was as inhibitory if not more inhibitory than the parent compound, as shown by the data presented in Figure 7. That the impermeant conjugate inhibited the oxidase suggests that the sulfonylurea binding site is at the external surface of the plasma membrane.

### Chloroaniline α-Cyclodextrin Conjugates

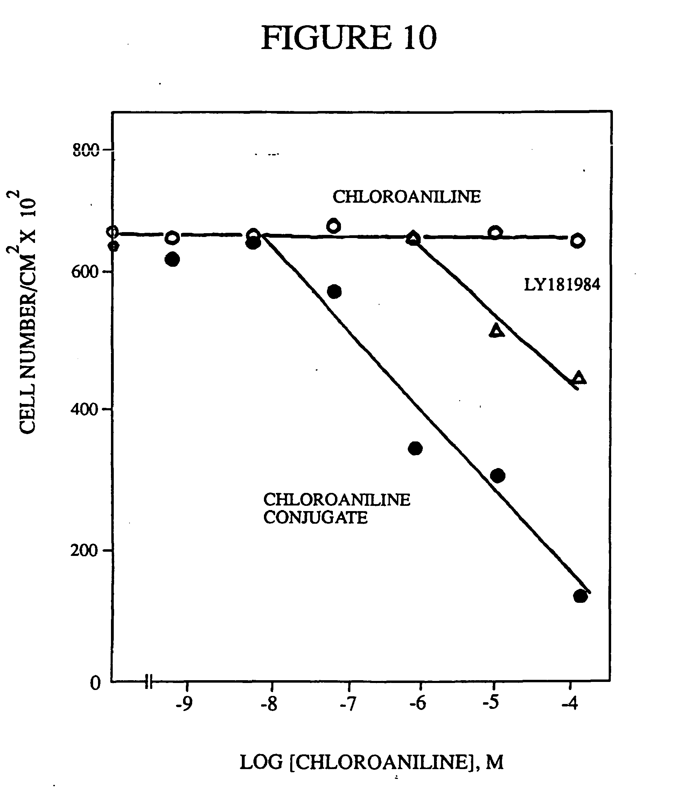

Chloroaniline, the B ring of sulfonylurea, was conjugated to α-cyclodextrin through a linking group as described for sulfonylurea. The conjugate inhibited the NADH oxidase activity from HeLa cell plasma membranes, while the free chloroaniline was inactive. The data is shown in Figure 10. The conjugate also inhibited the growth of HeLa cells more strongly than LY 181984, where the free chloroaniline was inactive. When tested for growth inhibition of non-transformed CHO cells, the conjugate did not inhibit growth or NADH oxidase activity.

### Capsaicin α-cyclodextrin Conjugates

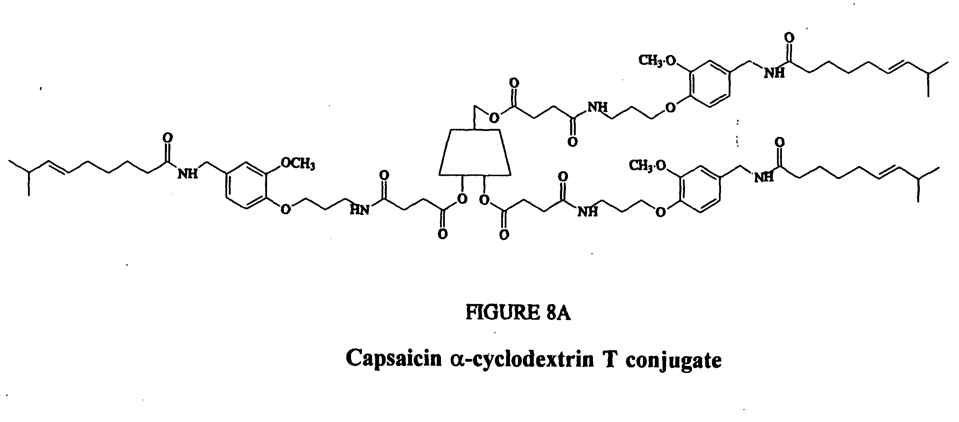

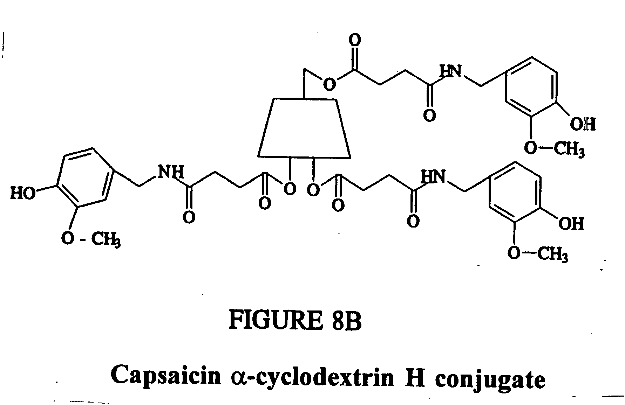

The capsaicin "head" and "tail" rings were conjugated to α-cyclodextrin as described for sulfonylurea. The structure of the tail conjugate is shown in Figure 8A, and the structure of the head conjugate is shown in Figure 8B.

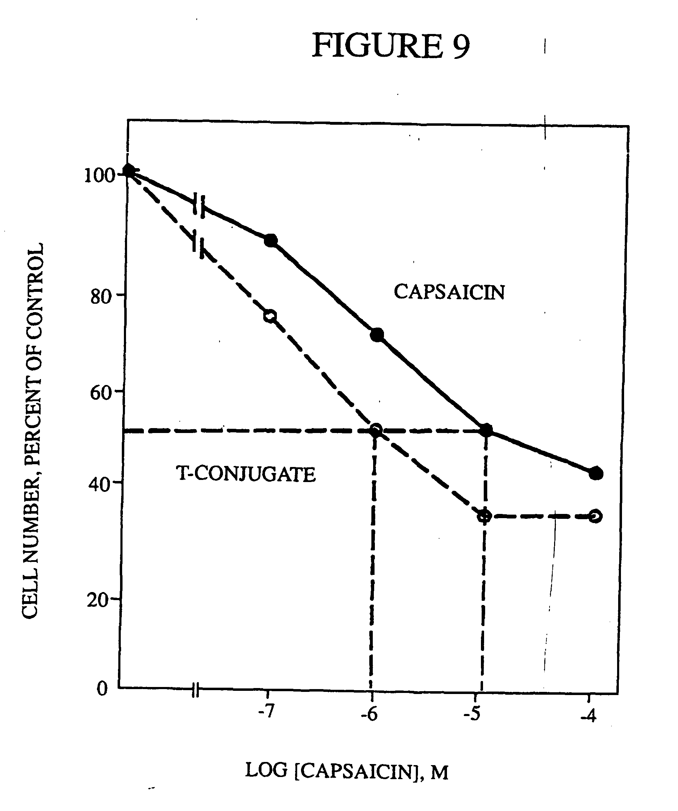

A growth inhibition assay comparing the ability of native capsaicin and the T conjugate to inhibit the growth of HeLa cells over a period of 72 hours. The data are shown in Figure 9. The concentration of drug able to inhibit growth by 50% was approximately 1 mM for the T conjugate, and greater than 10 mM for native capsaicin.

These data show that conjugates of an impermeant carrier molecule and an NADH oxidase inhibitor are able to inhibit plasma membrane NADH oxidase on tumor cells, and to slow the growth of tumor cells. The conjugate substantially increases the activity of several compounds, particularly sulfonylureas, chloroaniline, and vanillylmethylamine.

### Antibody Conjugates

Drug conjugates targeted to the NADH oxidase of virally-infected cells prevent the growth of these cells without effect on the growth of uninfected cells.

Antisera were generated to synthetic peptides from the deduced amino acid sequence of the major glycoprotein of feline immunodeficiency virus (FIV). Two were to the extracellular (surface) domain and two were to the cytoplasmic domain. The antisera were shown by Western blot analysis to be specific for the viral glycoprotein. Two (S-I and S-II, extracellular) were specific, while with L-I and L-II (cytoplasmic), L-I especially, cross-reacted with a host protein as well.

The antisera were conjugated to adriamycin through an activated carboxyl of the antibody to the amine of adriamycin free base using 2-isobutoxyl-1-isobutoxycarbonyl-1,2-dihydroxyquinoline, where the antibody was bound to an Ag-BSA sepharose affinity column. The concentration of drug conjugated was determined spectroscopically and was tested initially at a concentration of 0.2 µM on the growth of FIV-infected and -uninfected Crandall feline leukemia cells.

In the first experiment, with FIV-infected cells, growth was inhibited by both surface antigen antibody conjugates and by the conjugate of the antibody to L-I but not with L-II. Similar results were obtained in a second trial. Uninfected cells were not inhibited by the drug conjugates when tested at the same concentrations, except for the conjugate with the L-I which cross-reacted with a surface glycoprotein of normal cells. Unconjugated drug inhibited the growth of both normal and infected cells but only at concentrations of 10 µM or higher.

A dilution curve indicated that the conjugate of S-1 when tested with infected cells, was active even in the nanomolar range suggesting a margin of safety of about 10,000 for the drug conjugate (the concentration that stops the growth of infected cells compared to the concentration necessary to stop the growth of uninfected cells).

Adriamycin conjugate prepared as described above combined with plasma membrane vesicles was isolated from FIV-infected CFK cells. The conjugate was found to be 10-fold more effective than free adriamycin in inhibiting the activity of the NADH oxidase activity of the isolated plasma membranes.

The principal use of this is as a target for antiviral agents. In addition, it provides the basis for a new strategy of antiviral drug design where drugs are combined with antibodies to enhance efficacy and reduce unwanted toxicities. The conjugated drugs need not enter cells to be effective and can be targeted specifically to virus-infected cells or other types of cells carrying specific determinants (e.g., CD-4 T lymphocytes). This is an improvement over what now exists because it provides a cell surface target and a high degree of specificity. Drugs need not enter the cell to be effective. By targeting specifically to infected cells, only infected cells are killed and uninfected cells remain unharmed.

## Claims

1. A method for screening agents for their inhibitory effect on plasma membrane NADH oxidase derived from neoplastic cells, said method comprising:
combining in an assay medium said NADH oxidase with said agent in the presence of NADH;
measuring the rate of disappearance of NADH, disappearance of a reactant, or formation of a reaction product as a result of electron transport from said NADH oxidase; and
comparing the inhibitory effect on plasma membrane NADH oxidase derived from neoplastic cells to the inhibitory effect on plasma membrane NADH oxidase from normal cells, wherein said plasma membrane NADH oxidase derived from neoplastic cells and said plasma membrane NADH oxidase from normal cells are physically and functionally different, to identify an inhibitor which discriminates between NADH oxidase from normal cells and NADH oxidase from neoplastic cells.

2. A method according to claim 1, wherein said reactant is ascorbate free radical.

3. A method according to claim 1, wherein said NADH oxidase is present as part of a plasma membrane.

4. A method for determining neoplasia in a mammalian host, said method comprising:
detecting the presence of an NADH oxidase associated with neoplastic cells as compared to a physically and functionally different NADH oxidase associated with normal cells in a biological sample of said host by means of contacting a sample from the mammalian host with an antibody which specifically binds to the NADH oxidase of neoplastic cells but not to the NADH oxidase of normal cells, whereby neoplasia is determined in the mammalian host when said antibody binds to the sample.

5. A method for determining neoplasia in a mammalian host, said method comprising:
combining in an assay medium in the presence of NADH a physiological sample from said host comprising a plasma membrane NADH oxidase and an NADH oxidase inhibitor which discriminates between NADH oxidase from normal cells and NADH oxidase from neoplastic cells, wherein said NADH oxidase from normal cells and NADH oxidase from neoplastic cells are physically and functionally different; and
determining the level of said inhibitor at which said NADH oxidase in said sample is inhibited as compared to NADH oxidase from normal cells, where a lower level of inhibitor is indicative of neoplasia.

6. A method according to claim 5, wherein said determining is with ascorbate free radical as a reactant.

7. A method according to claim 5, wherein said NADH oxidase is present as part of a plasma membrane.

8. A method according to claim 7, wherein said inhibitor is an antineoplastic agent, and further comprising in a second assay adding an NADH oxidase stimulator to a second assay medium comprising a physiological sample from said host comprising a plasma membrane NADH oxidase in the presence of NADH; and determining the effect of said stimulator on NADH oxidase activity, wherein a lack of effect is indicative of neoplastic cells.

9. A method according to claim 4 or 5, wherein said physiological sample is a blood sample.

10. A method according to claim 4 or 5, wherein said physiological sample is a serum sample.

## Patentansprüche

1. Verfahren zum Screenen von Mitteln auf ihre inhibierende Wirkung auf Plasmamembran-NADH-Oxidase, die von neoplastischen Zellen stammt, wobei das Verfahren Folgendes umfasst:
das Kombinieren der NADH-Oxidase in einem Testmedium mit dem Mittel in Gegenwart von NADH;
das Messen der Geschwindigkeit des Verschwindens von NADH, des Verschwindens eines Reaktanten oder der Bildung eines Reaktionsprodukts als Resultat von Elektronentransport aus der NADH-Oxidase; sowie
das Vergleichen der inhibierenden Wirkung auf Plasmamembran-NADH-Oxidase, die von neoplastischen Zellen stammt, mit der inhibierenden Wirkung auf Plasmamembran-NADH-Oxidase normaler Zellen, worin sich die Plasmamembran-NADH-Oxidase, die von neoplastischen Zellen stammt, und die Plasmamembran-NADH-Oxidase normaler Zellen physisch und funktionell voneinander unterscheiden, um einen Inhibitor zu identifizieren, der zwischen NADH-Oxidase normaler Zellen und NADH-Oxidase neoplastischer Zellen unterscheidet.

2. Verfahren nach Anspruch 1, worin der Reaktant ein freies Ascorbat-Radikal ist.

3. Verfahren nach Anspruch 1, worin die NADH-Oxidase als Teil einer Plasmamembran vorliegt.

4. Verfahren zur Bestimmung von Neoplasie in einem Säugetierwirt, wobei das Verfahren Folgendes umfasst:
das Nachweisen der Gegenwart einer NADH-Oxidase, die mit neoplastischen Zellen assoziiert ist, im Vergleich zu einer physisch und funktionell unterschiedlichen NADH-Oxidase, die mit normalen Zellen assoziiert ist, in einer biologischen Probe des Wirts durch Kontaktieren einer Probe des Säugetierwirts mit einem Antikörper, der spezifisch an die NADH-Oxidase neoplastischer Zellen, aber nicht an die NADH-Oxidase normaler Zellen bindet, wodurch Neoplasie in dem Säugetierwirt bestimmt wird, wenn der Antikörper an die Probe bindet.

5. Verfahren zur Bestimmung von Neoplasie in einem Säugetierwirt, wobei das Verfahren Folgendes umfasst:
das Kombinieren einer physiologischen Probe des Wirts, die eine Plasmamembran-NADH-Oxidase umfasst, und eines NADH-Oxidaseinhibitors, der zwischen NADH-Oxidase normaler Zellen und NADH-Oxidase neoplastischer Zellen unterscheidet, in einem Testmedium in Gegenwart von NADH, wobei die NADH-Oxidase normaler Zellen und die NADH-Oxidase neoplastischer Zellen physisch und funktionell unterschiedlich sind; sowie
das Bestimmen jener Menge des Inhibitors, bei der die NADH-Oxidase in der Probe im Vergleich zu NADH-Oxidase normaler Zellen inhibiert wird, wobei eine geringere Menge des Inhibitors ein Indikator für Neoplasie ist.

6. Verfahren nach Anspruch 5, worin die Bestimmung mit freien Ascorbat-Radikalen als Reaktant erfolgt.

7. Verfahren nach Anspruch 5, worin die NADH-Oxidase als Teil einer Plasmamembran vorliegt.

8. Verfahren nach Anspruch 7, worin der Inhibitor ein antineoplastisches Mittel ist und das weiters in einem zweiten Test das Zusetzen eines NADH-Oxidase-Stimulators zu einem zweiten Testmedium, das eine physiologische Probe des Wirts umfasst, die eine Plasmamembran-NADH-Oxidase umfasst, in Gegenwart von NADH; sowie das Bestimmen der Wirkung des Stimulators auf die NADH-Oxidase-Aktivität umfasst, worin fehlende Wirkung ein Indikator für neoplastische Zellen ist.

9. Verfahren nach Anspruch 4 oder 5, worin die physiologische Probe eine Blutprobe ist.

10. Verfahren nach Anspruch 4 oder 5, worin die physiologische Probe eine Serumprobe ist.

## Revendications

1. Procédé pour cribler l'effet inhibiteur d'agents sur la NADH oxydase de membrane plasmique dérivée de cellules néoplasiques, ledit procédé comprenant les étapes consistant à:
combiner dans un milieu de dosage ladite NADH oxydase avec ledit agent en présence de NADH;
mesurer la vitesse de disparition du NADH, la disparition d'un réactif, ou la formation d'un produit de réaction par suite d'un transport d'électrons depuis ladite NADH oxydase; et
comparer l'effet inhibiteur sur la NADH oxydase de membrane plasmique dérivée de cellules néoplasiques à l'effet inhibiteur sur la NADH oxydase de membrane plasmique à partir de cellules normales, dans lequel ladite NADH oxydase de membrane plasmique dérivée de cellules néoplasiques et ladite NADH oxydase de membrane plasmique provenant de cellules normales sont physiquement et fonctionnellement différentes, pour identifier un inhibiteur qui distingue la NADH oxydase provenant de cellules normales de la NADH oxydase provenant de cellules néoplasiques.

2. Procédé selon la revendication 1, dans lequel ledit réactif est un radical libre ascorbate.

3. Procédé selon la revendication 1, dans lequel ladite NADH oxydase est présente en tant que partie d'une membrane plasmique.

4. Procédé pour déterminer une néoplasie chez un hôte mammifère, ledit procédé comprenant l'étape consistant à:
détecter la présence d'une NADH oxydase associée à des cellules néoplasiques comparée à une NADH oxydase physiquement et fonctionnellement différente associée à des cellules normales dans un échantillon biologique dudit hôte au moyen du contact d'un échantillon provenant de l'hôte mammifère avec un anticorps qui se lie spécifiquement à la NADH oxydase de cellules néoplasiques mais pas à la NADH oxydase de cellules normales, moyennant quoi la néoplasie est déterminée chez l'hôte mammifère lorsque ledit anticorps se lie à l'échantillon.

5. Procédé pour déterminer une néoplasie chez un hôte mammifère, ledit procédé comprenant les étapes consistant à:
combiner dans un milieu de dosage en présence de NADH un échantillon physiologique provenant dudit hôte comprenant une NADH oxydase de membrane plasmique et un inhibiteur de NADH oxydase qui distingue la NADH oxydase provenant de cellules normales de la NADH oxydase provenant de cellules néoplasiques, dans lequel ladite NADH oxydase provenant de cellules normales et ladite NADH oxydase provenant de cellules néoplasiques sont physiquement et fonctionnellement différentes; et
déterminer le taux dudit inhibiteur auquel ladite NADH oxydase dans ledit échantillon est inhibé comparé à la NADH oxydase provenant de cellules normales, où un taux inférieur d'inhibiteur indique une néoplasie.

6. Procédé selon la revendication 5, dans lequel ladite détermination se fait avec un radical libre ascorbate comme réactif.

7. Procédé selon la revendication 5, dans lequel ladite NADH oxydase est présente en tant que partie d'une membrane plasmique.

8. Procédé selon la revendication 7, dans lequel ledit inhibiteur est un agent anti-néoplasique, et comprenant en outre dans un second dosage, l'ajout d'un stimulateur de NADH oxydase à un second milieu de dosage comprenant un échantillon physiologique provenant dudit hôte comprenant une NADH oxydase de membrane plasmique en présence de NADH et la détermination de l'effet dudit stimulateur sur l'activité de NADH oxydase, dans lequel un manque d'effet indique des cellules néoplasiques.

9. Procédé selon la revendication 4 ou 5, dans lequel ledit échantillon physiologique est un échantillon de sang.

10. Procédé selon la revendication 4 ou 5, dans lequel ledit échantillon physiologique est un échantillon de sérum.
